# EUROPEAN PATENT SPECIFICATION

(11) **EP 3 816 294 B1**
(45) Date of publication and mention of the grant of the patent: **18.11.2026**
(21) Application number: 20197564.6
(22) Date of filing: 10.09.2016
(51) Int. Cl.: C12N 15/62, C12N 5/0781, C12N 5/0783, C07K 19/00, A61K 35/17, A61P 35/00

(54) **CHIMERIC ANTIGEN RECEPTORS AND USES THEREOF**
CHIMÄRE ANTIGENREZEPTOREN UND VERWENDUNGEN DAVON
RÉCEPTEURS D'ANTIGÈNES CHIMÉRIQUES ET LEURS UTILISATIONS

(30) Priority: 11.09.2015 AU 2015903719
(43) Date of publication of application: 05.05.2021
(62) Document number(s) of the earlier application(s) in accordance with Art. 76 EPC: 16843305.0 / 3 347 474
(73) Proprietor: Biosceptre (Aust) Pty Ltd, North Ryde, NSW 2113 (AU)
(72) Inventor: Coombs, Justin Taylor, Wattle Park, South Australia 5066 (AU); Barry, Simon Charles, Longwood, South Australia 5153 (AU); Sadlon, Timothy John, Maylands, South Australia 5069 (AU)
(74) Representative: Kuttenkeuler, David

(56) References cited:
- WO-A1-2011/020155
- WO-A1-2011/075789
- WO-A1-2012/079000
- WO-A1-2014/055657
- WO-A2-2012/082841
- US-A1- 2013 273 085
- JULIAN A. BARDEN ET AL: "Non-Functional P2X7: A Novel and Ubiquitous Target in Human Cancer", JOURNAL OF CLINICAL & CELLULAR IMMUNOLOGY, vol. 5, no. 4, 1 January 2014 (2014-01-01), pages 1 - 5, XP055367721, DOI: 10.4172/2155-9899.1000237
- STEPHAN MATTHIAS T ET AL: "T cell-encoded CD80 and 4-1BBL induce auto- and transcostimulation, resulting in potent tumor rejection", NATURE MEDICINE, NATURE PUB. CO, NEW YORK, vol. 13, no. 12, 1 December 2007 (2007-12-01), pages 1440 - 1449, XP002571429, ISSN: 1078-8956, [retrieved on 20071118], DOI: 10.1038/NM1676
- H. J. PEGRAM ET AL: "Tumor-targeted T cells modified to secrete IL-12 eradicate systemic tumors without need for prior conditioning", BLOOD, vol. 119, no. 18, 21 February 2012 (2012-02-21), pages 4133 - 4141, XP055109416, ISSN: 0006-4971, DOI: 10.1182/blood-2011-12-400044

## Description

### FIELD OF THE INVENTION

The present invention relates to chimeric antigen receptors, genetically modified cells expressing chimeric antigen receptors and use of said genetically modified cells for use in methods for killing cancer cells.

### BACKGROUND OF THE INVENTION

The immune system has highly evolved and specific mechanisms that protect us from a range of pathologies. Amongst these pathologies is the detection and elimination of unwanted pathogens such as bacterial infections, virally infected cells, and importantly, mutated cells that may cause malignant neoplasia (cancer). The ability for the immune system to prevent the formation and growth of cancers is dependent on the ability of the cells of the immune system to distinguish between a 'healthy' cell and a 'diseased' (e.g. neoplastic or pre-neoplastic) cell. This is achieved by recognition of cell markers (antigens) that are indicative of the transition in a cell from a healthy state to a diseased state.

There have been many attempts to develop immunotherapeutic approaches to treat cancer by manipulating or directing the immune system to target cells expressing cancer cell antigens. Immunotherapeutic approaches have largely centred on either exploiting the humoral immune system by utilising isolated or engineered antibodies or, more recently, the cellular arm of the immune system.

Early attempts to utilise cellular immunotherapy for the treatment of cancer utilised T lymphocytes isolated from tumours and expanded *ex vivo.* Whilst this approach has provided some initial promise in early investigations, there are many technical challenges associated with this approach. The ability to isolate and expand T cell populations to clinically relevant numbers is technically challenging and the poorly controlled nature of the expansion results in a final T cell population that is distinctly heterogeneous, and may contain only a small number of cancer antigen-specific T cells. As a result, the efficacy of this method is unpredictable and variable.

In order to address some of the shortfalls related to the use of *ex vivo* expanded tumour-isolated T cells, chimeric antigen receptors (CARs or artificial T cell receptors) began to be developed in the late 1980s. Chimeric antigen receptors are created by linking an extracellular region that is specific for a desired antigen to a signalling region, resulting in an antigen-specific receptor that can induce T cell function.

Transformation of isolated T cells with CARs results in a population of T cells that are specific for a given antigen. As a result, large populations of antigen-specific T cells can be generated and used for immunotherapy.

Initial clinical trials of CAR-transformed T cells specific for tumour associated antigens were promising. However, the efficacy of the CAR-transformed T cells led to significant hypercytokinemia, and ultimately death in some patients. These adverse effects are largely believed to be induced by on-target, but off-tumour activity of the CAR-transformed T cells induced as a result of endogenous expression of the cognate antigen for the CAR on healthy, non-cancerous, cell populations.

It is therefore apparent that there is a need for the development of a CAR that targets a tumour-associated antigen which is selectively expressed by cancerous cells but not endogenously expressed on non-cancerous cells.

WO 2012/079000 discloses use of chimeric antigen receptor-modified T cells to treat cancer.

WO 2014/055657 discloses use of a trans-signalling approach in chimeric antigen receptors.

WO 2011/075789 discloses antibodies to non-functional oligomeric P2X7 receptors.

WO 2011/020155 discloses anti P2X7 receptor antibodies and fragments thereof.

JULIAN A. BARDEN ET AL (in: "Non-Functional P2X7: A Novel and Ubiquitous Target in Human Cancer",JOURNAL OF CLINICAL & CELLULAR IMMUNOLOGY, vol. 5, no. 4, 1 January 2014), discloses that the nfP2X7 is expressed ubiquitously on the surface of cancer cells.

The discussion of documents, acts, materials, devices, articles and the like is included in this specification solely for the purpose of providing a context for the present invention. It is not suggested or represented that any or all of these matters formed part of the prior art base or were common general knowledge in the field relevant to the present invention as it existed before the priority date of each claim of this application.

### SUMMARY OF THE INVENTION

The present invention is predicated in part on the recognition that due to the significant 'on-target' but 'off-tumour' activity of CAR-expressing immune cells, there is a need for the development of a CAR, and a genetically modified cell expressing the same, which targets a marker specifically associated with a range of neoplastic (cancerous) or pre-neoplastic (pre-cancerous) cells. The inventors have recognised that a dysfunctional P2X₇ receptor is a suitable marker for targeting with a CAR.

Accordingly, in a first aspect, the present invention provides a chimeric antigen receptor including: an antigen-recognition domain and a signalling domain, wherein the antigen-recognition domain recognises a dysfunctional P2X₇ receptor, wherein the antigen-recognition domain recognises an epitope that includes amino acids 200 to 210 of the dysfunctional P2X₇ receptor (corresponding to amino acids 200 to 210 of SEQ ID NO: 3); a transmembrane domain; and a signalling domain including an intracellular signalling portion of an activation receptor and/or an intracellular signalling portion of a co-stimulatory receptor.

In some embodiments, the antigen-recognition domain recognises an epitope associated with an adenosine triphosphate (ATP)-binding site of the dysfunctional P2X₇ receptor. In some embodiments, the dysfunctional P2X₇ receptor has a reduced capacity to bind ATP at the ATP-binding site compared to an ATP-binding capacity of a wild-type (functional) P2X₇ receptor. In some embodiments the dysfunctional P2X₇ receptor cannot bind ATP at the ATP-binding site.

In some embodiments, the dysfunctional P2X₇ receptor has a conformational change that renders the receptor dysfunctional. In some embodiments, the conformational change is a change of an amino acid from the trans-conformation to the cis-conformation. In some embodiments, the amino acid that has changed from a trans-conformation to a cis-conformation is proline at amino acid position 210 of the dysfunctional P2X₇ receptor.

In some embodiments, the antigen-recognition domain recognises an epitope that includes the proline at amino acid position 210 of the dysfunctional P2X₇ receptor. In some embodiments, the antigen-recognition domain recognises an epitope that includes one or more amino acid residues spanning from glycine at amino acid position 200 to cysteine at amino acid position 216, inclusive, of the dysfunctional P2X₇ receptor.

The antigen-recognition domain of the CAR can be any suitable molecule that can interact with and specifically recognise a dysfunctional P2X₇ receptor, wherein the antigen-recognition domain recognises an epitope that includes amino acids 200 to 210 of the dysfunctional P2X₇ receptor (corresponding to amino acids 200 to 210 of SEQ ID NO: 3). However, in some embodiments, the antigen-recognition domain includes amino acid sequence homology to the amino acid sequence of an antibody, or a fragment thereof, that binds to the dysfunctional P2X₇ receptor. In some embodiments, the antigen-recognition domain includes amino acid sequence homology to the amino acid sequence of a fragment-antigen binding (Fab) portion of an antibody that binds to a dysfunctional P2X₇ receptor. In some embodiments, the antibody is a humanised antibody.

In some embodiments, the antigen-recognition domain includes amino acid sequence homology to the amino acid sequence of a single-chain variable fragment (scFv) or a multivalent scFv that binds to a dysfunctional P2X₇ receptor. In some embodiments, the multivalent scFv is a di-valent or tri-valent scFv.

In some embodiments, the antigen-recognition domain includes amino acid sequence homology to a single-antibody domain (sdAb) that binds to a dysfunctional P2X₇ receptor.

In some embodiments, the antigen recognition domain includes three CDRs of the sequences spanning positions 30 to 35, 50 to 67 or 98 to 108 of the sequences set forth in SEQ ID NOS: 10, 32, 33 or 34. In some embodiments, the antigen recognition domain includes three CDRs of the sequences set forth in SEQ ID NOS: 10, 32, 33 or 34.

In some embodiments, the signalling domain includes a portion derived from an activation receptor. In some embodiments, the activation receptor is a member of the CD3 co-receptor complex or is an Fc receptor. In some embodiments, the portion derived from the CD3 co-receptor complex is CD3-ζ. In some embodiments, the portion derived from the Fc receptor is FcεRI or FcγRI.

In some embodiments, the signalling domain includes a portion derived from a co-stimulatory receptor. In some embodiments, the signalling domain includes a portion derived from an activation receptor and a portion derived from a co-stimulatory receptor. In some embodiments, the co-stimulatory receptor is selected from the group consisting of CD27, CD28, CD30, CD40, DAP10, OX40, 4-1BB (CD137) and ICOS.

In a second aspect, the present invention provides a nucleic acid molecule including a nucleotide sequence encoding the chimeric antigen receptor according to the first aspect of the invention.

In a third aspect, the present invention provides a viral vector including a nucleic acid according to the second aspect, for viral transduction of a host cell..

In a fourth aspect, the present invention provides a genetically modified cell that includes a CAR according to the first aspect of the invention. In some embodiments, the cell includes two or more different CARs.

In a fifth aspect, the present invention provides a genetically modified cell that includes a nucleic acid molecule according to the second aspect of the invention, or a nucleic acid construct according to the third aspect of the invention, or a genomically integrated form of the construct. In some embodiments, the nucleic acid molecule or the nucleic acid construct encodes two or more different CARs.

In some embodiments of the fourth and fifth aspects of the invention, the two or more different CARs have different signalling domains.

In some embodiments of the fourth and fifth aspects of the invention, the cell includes a first CAR with a signalling domain including a portion derived from an activation receptor and a second CAR with a signalling domain including a portion derived from a co-stimulatory receptor. In some embodiments, the activation receptor is a member of the CD3 co-receptor complex or is an Fc receptor. In some embodiments, the co-stimulatory receptor is selected from the group consisting of CD27, CD28, CD30, CD40, DAP10, OX40, 4-1BB (CD137) and ICOS.

In some embodiments of the fourth and fifth aspects of the invention, the cell is further modified so as to constitutively express co-stimulatory receptors. In some embodiments, the cell is further modified so as to express ligands for the co-stimulatory receptors, thereby facilitating auto-stimulation of the cell.

In some embodiments of the fourth and fifth aspects of the invention, the cell is further modified to secrete cytokines. In some embodiments, the cytokines are selected from the group consisting of IL-2, IL- 7, IL-12, IL-15, IL-17 and IL-21, or a combination thereof.

In some embodiments of the fourth and fifth aspects of the invention, the cell is a leukocyte. In some embodiments, the cell is a Peripheral Blood Mononuclear Cell (PBMC), a lymphocyte, a T cell (including a CD4+ T cell or a CD8+ T cell), a natural killer cell, or a natural killer T cell.

In a sixth aspect, the present invention provides a genetically modified cell according to the fourth or fifth aspects of the invention for use in a method of killing a cell expressing a dysfunctional P2X₇ receptor, the method including exposing the cell expressing a dysfunctional P2X₇ receptor to the genetically modified cell.

In some embodiments of the sixth aspect of the invention, the CAR directly recognises the dysfunctional P2X₇ receptor, or recognises the dysfunctional P2X₇ receptor via an intermediate. In some embodiments, the intermediate is a probe that binds to a dysfunctional P2X₇ receptor and the CAR recognises the probe. In some embodiments, the probe is an antibody or an aptamer. In some embodiments, the probe includes a tag and the CAR recognises the tag.

In some embodiments of the sixth aspect of the invention, the CAR directly recognises the dysfunctional P2X₇ receptor, or recognises the dysfunctional P2X₇ receptor via an intermediate. In some embodiments, the intermediate is a probe that binds to a dysfunctional P2X₇ receptor and the CAR recognises the probe. In some embodiments, the probe is an antibody or an aptamer. In some embodiments, the probe includes a tag and the CAR recognises the tag.

In some embodiments of the sixth aspect of the invention, the cell expressing a dysfunctional P2X₇ receptor is exposed to the genetically modified cell together with an exogenous cytokine. In some embodiments, the genetically modified cell is a genetically modified cell, autologous to the cell expressing a dysfunctional P2X₇ receptor.

In some embodiments of the sixth aspect of the invention, the cell expressing a dysfunctional P2X₇ receptor is a cancer cell. In some embodiments the cancer is selected from the group consisting of; brain cancer, oesophageal cancer, mouth cancer, tongue cancer, thyroid cancer, lung cancer, stomach cancer, pancreatic cancer, kidney cancer, colon cancer, rectal cancer, prostate cancer, bladder cancer, cervical cancer, epithelial cell cancers, skin cancer, leukaemia, lymphoma, myeloma, breast cancer, ovarian cancer, endometrial cancer and testicular cancer. In some embodiments the cancer is selected from the group consisting of; lung cancer, oesophageal cancer, stomach cancer, colon cancer, prostate cancer, bladder cancer, cervical cancer, vaginal cancers, epithelial cell cancers, skin cancer, blood-related cancers, breast cancer, endometrial cancer, uterine cancer and testicular cancer.

In some embodiments of the sixth aspect of the invention, the cancer is metastatic. In some embodiments, the cancer is stage III cancer or is stage IV cancer.

In an seventh aspect, the present invention provides a pharmaceutical composition including a genetically modified cell according to the fourth or fifth aspects of the invention and a pharmaceutically acceptable carrier. In some embodiments, the pharmaceutical composition includes suitable adjuvants which may consist of cytokines. In some embodiments, the pharmaceutical composition may also include an intermediate as described herein.

### BRIEF DESCRIPTION OF THE FIGURES

For a further understanding of the aspects and advantages of the present invention, reference should be made to the following detailed description, taken in conjunction with the accompanying drawings.
**FIGURE 1** - a schematic showing the arrangement of an anti-non-functional (nf) P2X₇ receptor chimeric antigen receptor (CAR) according to an embodiment of the present invention.
**FIGURE 2** - a schematic showing the BLIV plasmid used for expression of the anti-nf P2X7 receptor CAR in Figure 1.
**FIGURE** 3 - an electrophoresis gel showing restriction fragments from *Bam*HI restricted DNA isolated from *E.coli* clones transformed with the BLIV plasmid.
**FIGURE 4** - an electrophoresis gel showing restriction fragments from *Eco*RI, *Bam*HI and *Pst*I restricted DNA isolated from selected *E.coli* clones transformed with the BLIV plasmid.
**FIGURE 5** - shows microscopy images of 293T cells transfected with plasmids required for the construction of lentiviral vectors containing the BLIV-CAR-short hinge construct and 293T cells transduced with supernatant containing the lentiviral vectors.
**FIGURE 6** - shows microscopy images of 293T cells transfected with plasmids required for the construction of lentiviral vectors containing the BLIV-CAR-long hinge construct and 293T cells transduced with supernatant containing the lentiviral vectors.
**FIGURE 7** - FACS analysis of the cell purity of T cells purified with the RosetteSep human CD8+ T cell enrichment kit.
**FIGURE 8** - FACS analysis of killing assays comprising the co-culturing of CD8+ T cells and BT549 cells.
**FIGURE 9** - Graph illustrating the percentage of dye-labelled target cells deleted after 48hr of co-culture with CD8+ T cells transduced with lentiviral vectors containing the BLIV-CAR-short hinge and BLIV-CAR-long hinge plasmids compared to non-transduced CD8+ T cells and CD8+ T cells transduced with empty BLIV plasmids.
**FIGURE 10** - Alignment of the PEP2-2-1-1, PEP2-472-2 and PEP2-2-12 binding peptides with antibodies directed against the nf-P2X₇ receptor.
**FIGURE 11** - A schematic showing the arrangement of an anti-nf P2X₇ receptor CAR according to a further embodiment of the present invention.
**FIGURE 12** - A schematic showing the pCDH plasmid used for expression of an anti-nf P2X₇ receptor CAR of Figure 11.
**FIGURE 13** - An electrophoresis gel showing restriction fragments from *Eco*RI and *Not* I restricted DNA isolated from selected Sure 2 clones, transformed with the pCDH plasmid.
**FIGURE 14** - FACS analysis of the efficiency of transfection of HEK293T cells.
**FIGURE 15** - Representative histograms of FACS analysis of lentiviral transduction efficiency.
**FIGURE 16** - FACS analysis of the percentage of transduced CD8 cells expressing GFP.
**FIGURE 17** - Illustration of the backbone of the fusion protein for generation of non-functional and functional P2X₇ receptors.
**FIGURE 18** - An electrophoresis gel showing restriction fragments from *Bam HI* and *Pmel* restricted DNA isolated from selected *E.clonf*^{®}10G clones transformed with EXD2_K193A or EXD2_WT containing pDONR-107 vectors.
**FIGURE 19** - An electrophoresis gel showing restriction fragments from *Bam HI* restricted DNA isolated from selected *E.cloni*^{®}10G clones transformed with EXD2_K193A or EXD2_WT containing pLV-416 vectors.
**FIGURE 20** - FACS analysis of transduction of lentiviral packaging of HEK293 cells with pLV-416- EXD2_K193A and pLV-416-EXD2_WT.
**FIGURE 21** - FACS analysis of transduction of HEK293 with lentivirus containing either pLV-416- EXD2_K193A or pLV-416-EXD2_WT constructs.
**FIGURE 22** - Graph illustrating the killing of nfP2X₇ expressing HEK target cells and 231 breast cancer cell by T cells expressing PEP2-2-1-1, PEP2-472-2 CARs

### DETAILED DESCRIPTION OF THE INVENTION

The nucleotide and polypeptide sequences referred to herein are represented by a sequence identifier number (SEQ ID NO:). A summary of the sequence identifiers is provided in Table 1. A sequence listing is also provided at the end of the specification.

**TABLE 1**

| **Summary of Sequence Identifiers** | |
|---|---|
| **Sequence Identifier** | **Sequence** |
| SEQ ID NO: 1 | Human P2X₇ receptor mRNA sequence |
| SEQ ID NO: 2 | Human P2X₇ receptor coding (cDNA) sequence |
| SEQ ID NO: 3 | Human P2X₇ receptor amino acid sequence |
| SEQ ID NO: 4 | CD3ζ chain amino acid sequence |
| SEQ ID NO: 5 | CD3ε chain amino acid sequence |
| SEQ ID NO: 6 | CD3γ chain amino acid sequence |
| SEQ ID NO: 7 | CD3δ chain amino acid sequence |
| SEQ ID NO: 8 | FcεR1 amino acid sequence |
| SEQ ID NO: 9 | FcγRI amino acid sequence |
| SEQ ID NO: 10 | PEP2-2-3 amino acid sequence |
| SEQ ID NO: 11 | PEP2-2-3 nucleotide sequence |
| SEQ ID NO: 12 | CD8a signalling amino acid sequence |
| SEQ ID NO: 13 | CD8a signalling nucleotide sequence |
| SEQ ID NO: 14 | Long hinge amino acid sequence |
| SEQ ID NO: 15 | Long hinge nucleotide sequence |
| SEQ ID NO: 16 | Short hinge amino acid sequence |
| SEQ ID NO: 17 | Short hinge nucleotide sequence |
| SEQ ID NO: 18 | Amino acid sequence of a portion of the CD28 co-stimulatory receptor |
| SEQ ID NO: 19 | Nucleotide sequence coding for SEQ ID NO: 18 |
| SEQ ID NO: 20 | Amino acid sequence of a portion of the OX40 co-stimulatory receptor |
| SEQ ID NO: 21 | Nucleotide sequence coding for SEQ ID NO: 20 |
| SEQ ID NO: 22 | Amino acid sequence of a portion of the CD3 zeta co-receptor complex |
| SEQ ID NO: 23 | Nucleotide sequence coding for SEQ ID NO: 22 |
| SEQ ID NO: 24 | P2A amino acid sequence |
| SEQ ID NO: 25 | P2A nucleotide sequence |
| SEQ ID NO: 26 | PEP2-2-3 binding peptide CAR amino acid sequence - long hinge |
| SEQ ID NO: 27 | PEP2-2-3 binding peptide CAR amino acid sequence - short hinge |
| SEQ ID NO: 28 | PEP2-2-3 binding peptide CAR nucleotide sequence - long hinge |
| SEQ ID NO: 29 | PEP2-2-3 binding peptide CAR nucleotide sequence - short hinge |
| SEQ ID NO: 30 | Human CD8 leader amino acid sequence |
| SEQ ID NO: 31 | Human CD8 nucleotide sequence |
| SEQ ID NO: 32 | Amino acid sequence of PEP2-2-1-1 binding peptide |
| SEQ ID NO: 33 | Amino acid sequence of PEP2-472-2 binding peptide |
| SEQ ID NO: 34 | Amino acid sequence of PEP2-2-12 binding peptide |
| SEQ ID NO: 35 | Nucleotide sequence of PEP2-2-1-1 CAR |
| SEQ ID NO: 36 | Nucleotide sequence of PEP2-472-2 CAR |
| SEQ ID NO: 37 | Nucleotide sequence of PEP2-2-12 CAR |
| SEQ ID NO: 38 | pCHD-CMV-For primer |
| SEQ ID NO: 39 | pCHD-coGFP-Rev primer |
| SEQ ID NO: 40 | 2-2-1-1-Rev primer |
| SEQ ID NO: 41 | 2-2-1-1-For primer |
| SEQ ID NO: 42 | 2-472-2-Rev primer |
| SEQ ID NO: 43 | 2-472-2-For primer |
| SEQ ID NO: 44 | 2-12-2-Rev primer |
| SEQ ID NO: 45 | Com-For-1 primer |
| SEQ ID NO: 46 | Com-For-2 primer |
| SEQ ID NO: 47 | EXD2_K193A gene block |
| SEQ ID NO: 48 | EXD2_WT gene block |
| SEQ ID NO: 49 | EXD-F1 primer |
| SEQ ID NO: 50 | EXD2-R1 primer |
| SEQ ID NO: 51 | EXD2-F1 primer |
| SEQ ID NO: 52 | Amino acid sequence of PEP2-2-1-1 CAR |
| SEQ ID NO: 53 | Amino acid sequence of PEP2-472-2 CAR |
| SEQ ID NO: 54 | Amino acid sequence of PEP2-2-12 CAR |

The inventors have recognized that due to the significant 'on-target' but 'off-tumour' activity of chimeric antigen receptor (CAR) expressing immune cells, there is a need for the development of a CAR, and a genetically modified cell expressing the same, which targets a marker specifically associated with neoplastic (cancerous) or pre-neoplastic (pre-cancerous) cells. The inventors have recognised that a dysfunctional P2X₇ receptor is a suitable marker for targeting with a CAR expressing immune cell, in a range of cancers.

Accordingly, in a first aspect, the present invention provides a chimeric antigen receptor (CAR) which includes an antigen-recognition domain, wherein the antigen-recognition domain recognises an epitope that includes amino acids 200 to 210 of the dysfunctional P2X₇ receptor (corresponding to amino acids 200 to 210 of SEQ ID NO: 3); a transmembrane domain; and a signalling domain including an intracellular signalling portion of an activation receptor and/or an intracellular signalling portion of a co-stimulatory receptor..

Chimeric antigen receptors are artificially constructed proteins that upon expression on the surface of a cell can induce an antigen-specific cellular response. A CAR includes at least two domains; the first domain being an antigen-recognition domain that specifically recognises an antigen, or more specifically an epitope portion, or portions, of an antigen; and the second domain being a signalling domain that is capable of inducing, or participating in the induction, of an intracellular signalling pathway.

The combination of these two domains determines the antigen specificity of the CAR and the ability of the CAR to induce a desired cellular response, the latter of which is also dependent on the host cell of the CAR. For example, the activation of a CAR expressed in a T-helper cell and having a signalling domain comprising a CD3 activation domain, may, once activated by encountering its cognate antigen, induce the CD4+ T-helper cell to secrete a range of cytokines. In a further example, the same CAR when expressed in a CD8+ cytotoxic T cell, once activated by a cell expressing the cognate antigen, may induce the release of cytotoxins that ultimately lead to the induction of apoptosis of the antigen-expressing cell.

In addition to the antigen-recognition domain and the signalling domain a CAR may further include additional components, or portions. For example, the CAR may include a transmembrane domain which may comprise a portion of, or may be associated with, the signalling domain of the CAR. The transmembrane domain is typically one or more hydrophobic helices, which spans the lipid bilayer of a cell and embeds the CAR within the cell membrane. The transmembrane domain of the CAR can be one determinant in the expression pattern of the CAR when associated with a cell. For example, using a transmembrane domain associated with a CD3 co-receptor can permit expression of the CAR in naïve T cells, whilst use of a transmembrane domain from a CD4 co-receptor may direct expression of a CAR in T-helper cells but not cytotoxic T cells.

A further component or portion of a CAR may be a linker domain. The linker domain (also known as the spacer or hinge domain) may span from the extracellular side of the transmembrane domain to the antigen-recognition domain, thereby linking the antigen-recognition domain to the transmembrane domain. Whilst in some cases a linker domain is not required for a functional CAR (i.e. the antigen-recognition domain can be connected directly to the transmembrane domain) in some circumstances the use of a linker domain allows for greater efficacy of the CAR. The linker domain can have a variety of functions including allowing flexibility of the CAR to permit the necessary orientation of the antigen-recognition domain of the CAR for binding to an antigen. Consequently, the linker domain can be any amino acid sequence that performs this function. One non-limiting example of a linker domain is a domain having amino acid sequence homology to the hinge region of an IgG antibody, such as the IgG1 hinge region. Alternative examples include amino acid sequences having sequence homology to the CH₂CH₃ region of an antibody or portions of the CD3 co-receptor complex, the CD4 co-receptor or the CD8 co-receptor.

The P2X₇ receptor (purinergic receptor P2X, ligand-gated ion channel, 7) is an ATP-gated ion channel that is expressed in a number of species including humans. The receptor is encoded by a gene, the official symbol of which is represented by *P2RX7.* The gene has also been referred to as P2X purinoceptor 7, ATP receptor, P2Z receptor, P2X7 receptor, and purinergic receptor P2X7 variant A. For the purposes of the present disclosure, the gene and encoded receptor will be referred to herein as *P2X7* and P2X₇, respectively.

The mRNA, coding (cDNA), and amino acid sequences of the human *P2X7* gene are set out in SEQ ID NOs: 1 to 3, respectively. The mRNA and amino acid sequences of the human *P2X7* gene are also represented by GenBank Accession Numbers NM_002562.5 and NP_002553.3, respectively. The *P2X7* gene is conserved in chimpanzee, Rhesus monkey, dog, cow, mouse, rat, pig, chicken, zebrafish, and frog. Further details of the *P2X7* gene in human and other species may be accessed from the GenBank database at the National Centre for Biotechnology Information (NCBI) (www.ncbi.nlm.nih.gov). For example, the Gene ID number for human *P2X7* is 5027, for chimpanzee is 452318, for monkey is 699455, for dog is 448778, for cow is 286814, for mouse is 18439, for zebrafish is 387298, and for frog is 398286. Furthermore, at least 73 organisms have orthologs with the human *P2X7* gene.

Further details regarding the *P2X7* gene in human and other species can also be found at the UniGene portal of the NCBI (for example see UniGene Hs. 729169 for human P2X₇-http://www.ncbi.nlm.nih.gov/UniGene/clust.cgi?UGID=4540770&TAXID=9606&SEARCH). Alternatively, details of the nucleotide and amino acid sequences for the *P2X7* gene can be accessed from the UniProt database (www.uniprot.org) wherein the UniProt ID for the human *P2X7* gene is Q99572. The contents of the GenBank and UniProt records are incorporated herein by reference.

The P2X₇ receptor is formed from three protein subunits (monomers), wherein in the native receptor in humans at least one of the monomers has an amino acid sequence set forth in SEQ ID NO: 3. It is to be understood that a "P2X₇ receptor" as referred to herein also includes naturally occurring variations of the receptor including splice variants, naturally occurring truncated forms and allelic variants of the receptor. A P2X₇ receptor may also include subunits that have a modified amino acid sequence, for example those including truncations, amino acid deletions or modifications of the amino acid set forth in SEQ ID NO: 3.

A "variant" of the *P2X7* gene or encoded protein may exhibit a nucleic acid or an amino acid sequence, respectively, that is at least 80% identical, at least 90% identical, at least 95% identical, at least 98% identical, at least 99% identical, or at least 99.9% identical to a native P2X₇ receptor, for example.

The P2X₇ receptor is activated by the binding of ATP to the ATP-binding site of the receptor. This leads to the rapid opening (within milliseconds) of a channel that selectively allows for movement of small cations across the membrane. After a short period of time (within seconds) a large pore is formed in the membrane of a cell that allows for permeation of the cell membrane by molecules up to 900 Da in size. This pore formation ultimately leads to depolarization of the cell and in many cases cytotoxicity and cell death. This role leads to a belief that the P2X₇ receptor is involved in apoptosis in a variety of cell types.

Like other molecules involved in apoptosis, such a Bcl2 and Bax, a decrease or loss in function of the P2X₇ receptor can lead to a cell that is comparatively resistant to induced apoptosis. In many cases this resistance to apoptosis is critical in the transition of a normal 'healthy' cell to a mutated pre-cancerous or cancerous cell. Consequently, the ability to target cells that have a decreased function, or a loss of function, of the P2X₇ receptor provides a promising target for cancer therapy.

Accordingly, in the first aspect of the invention the CAR recognises a dysfunctional P2X₇ receptor, wherein the antigen-recognition domain recognises an epitope that includes amino acids 200 to 210 of the dysfunctional P2X₇ receptor (corresponding to amino acids 200 to 210 of SEQ ID NO: 3). As used throughout the specification the term "dysfunctional" with reference to the P2X₇ receptor includes a decrease in function of the receptor with respect to its comparative function in a normal non-tumour cell. In some embodiments, the function of P2X₇ receptor may be decreased by at least 1%, 5%, 10%, 20%, 30%, 40%, 50%, 60%, 70%, 80%, 90% or greater than 99%. In some embodiments, the term "dysfunctional" may include a P2X₇ receptor that is non-functional. That is to say that the P2X₇ receptor is unable to be induced to permit permeability of cations and other molecules across the cell membrane.

Any change in the wild-type or native form of the P2X₇ receptor that leads to a dysfunctional receptor is encompassed herein. For example, the dysfunctional receptor may be the result of a mutation or alteration in one or more amino acids of the receptor that are associated with ATP binding to the receptor. In effect, the P2X₇ receptor is dysfunctional as it has a reduced capacity to, or cannot, bind ATP at the ATP-binding site. In this instance, the antigen-recognition domain of the chimeric antigen receptor will recognise an epitope of the dysfunctional P2X₇ receptor associated with the ATP-binding site. Consequently, in some embodiments of the first aspect of the invention, the antigen-recognition domain of the chimeric antigen receptor recognises an epitope of the dysfunctional P2X₇ receptor associated with the ATP-binding site. In some embodiments, the dysfunctional P2X₇ receptor has a reduced capacity to bind ATP compared to an ATP-binding capacity of a wild-type (functional) P2X₇ receptor. In some embodiments the dysfunctional P2X₇ receptor cannot bind ATP.

An alteration in one or more amino acids of the P2X₇ receptor may include a conformational change in one or more amino acids of the receptor. Therefore, in some embodiments of the first aspect of the invention the chimeric antigen receptor binds to a dysfunctional P2X₇ receptor having a conformational change that renders the receptor dysfunctional. Specifically, this conformational change may be a change in one or more amino acids of the P2X₇ receptor from a trans-conformation to a cis-conformation. In some embodiments, a proline at position 210 of the P2X₇ receptor changes from a trans-conformation to a cis-conformation. In this instance, the antigen-recognition domain of the CAR may recognise an epitope that includes proline at amino acid position 210 of the P2X₇ receptor. In some embodiments of the first aspect of the present invention, the antigen-recognition domain recognises an epitope that includes one or more amino acids spanning from glycine at amino acid position 200 to cysteine at amino acid position 216 (inclusive) of the dysfunctional P2X₇ receptor. In some embodiments of the first aspect of the present invention, the antigen-recognition domain recognises an epitope that includes the proline at position 210 of the dysfunctional P2X₇ receptor, and one or more of the amino acid residues spanning from glycine at amino acid position 200 to cysteine at amino acid position 216 (inclusive) of the dysfunctional P2X₇ receptor.

Whilst not wanting to be bound by theory, as a result of the conformational change of the proline at position 210 of the P2X₇ receptor, the three-dimensional structure of the receptor may be altered. This alteration in the three-dimensional structure may allow the antigen-recognition domain of the CAR to bind to amino acids, or epitopes, previously inaccessible in the native three-dimensional structure of the P2X₇ receptor. Therefore, in some embodiments the CAR recognises one or more epitopes of the P2X₇ receptor exposed to the antigen-recognition domain as a result of a trans- to cis-conformational change of the proline at position 210 of SEQ ID NO: 3. These epitopes may include one or more of the amino acids at position 200 to 210, or positions 297 to 306, inclusive, of the P2X₇ receptor. Accordingly, in some embodiments of the first aspect of the present invention, the antigen-recognition domain recognises an epitope that includes one or more of the amino acids at positions 200 to 210 and/or 297 to 306 of the P2X₇ receptor.

As used throughout the specification the term "recognises" relates to the ability of the antigen-recognition domain to associate with a dysfunctional P2X₇ receptor, a portion thereof, or an epitope thereof. In some embodiments, the antigen-recognition domain may directly bind to the dysfunctional P2X₇ receptor, or an epitope thereof. In other embodiments, the antigen-recognition domain may bind to a processed form of the dysfunctional P2X₇ receptor. As used in this context the term "processed form" relates to forms of the P2X₇ receptor which have been truncated or digested as a result of intracellular processing. Consequently, the recognition of the "processed form" of the dysfunctional P2X₇ receptor may be as a result of being presented in association with a major histocompatibility complex (MHC).

The antigen-recognition domain can be any suitable domain that can recognise a dysfunctional P2X₇ receptor, or epitope thereof, wherein the antigen-recognition domain recognises an epitope that includes amino acids 200 to 210 of the dysfunctional P2X₇ receptor (corresponding to amino acids 200 to 210 of SEQ ID NO: 3). As used throughout the specification the term "antigen-recognition domain" refers to the portion of the CAR that provides the specificity of the CAR for the dysfunctional P2X₇ receptor. The antigen-recognition domain may be all of, or may merely be part of, the extracellular region of the CAR. Suitable antigen-recognition domains, include, but are not limited to, polypeptides having sequence homology to the antigen-binding site of an antibody, or fragment thereof, that bind to a dysfunctional P2X₇ receptor. Therefore, in some embodiments of the first aspect of the invention, the antigen-recognition domain includes an amino acid sequence having homology to an antibody, or a fragment thereof, that binds to a dysfunctional P2X₇ receptor. In some embodiments, a portion of the antigen-recognition domain includes an amino acid sequence having homology to an antibody, or a fragment thereof, that binds to the dysfunctional P2X₇ receptor. The source homologous antibody sequence can be any suitable sequence of an antibody that has an affinity for the P2X₇ receptor. For example the sequence can share sequence homology with an antibody originating from one or more of the following species; human, non-human primate, mouse, rat, rabbit, sheep, goat, ferret, canine, chicken, feline, guinea pig, hamster, horse, cow, or pig. The antigen-recognition domain may share sequence homology with the sequence of a monoclonal antibody produced from a hybridoma cell line. When the originating species of the homologous antibody sequence is not human, the antibody is preferably a humanised antibody. The homologous antibody sequence may also be from a non-mammalian animal species such as a cartilaginous fish (e.g. shark IgNAR antibodies - see WO2012/073048). Alternatively, the antigen binding domain may include a modified protein scaffolds that provide functionality similar to shark antibodies, such as i-bodies which have binding moieties based on shark IgNAR antibodies (see WO2005/118629). Additionally, the antigen-recognition domain could be, could be derived from, or could share sequence homology with any other suitable binding molecule or peptide that can selectively interact with a dysfunctional P2X₇ receptor with an affinity sufficient to activate the CAR signalling domain. Methods are known in the art for the identification of antigen-binding proteins such as, *inter alia,* panning phage display libraries, protein affinity chromatography, co-immunoprecipitation and yeast two-hybrid systems (see Srinivasa Rao, V. et al. Int J Proteomics, 2014; article ID 147648).

In some embodiments the antigen-recognition domain of the CAR isa fragment-antigen binding (Fab) portion of an antibody that binds to a dysfunctional P2X₇ receptor. As will be understood in the art, a Fab portion of an antibody in composed of one constant region and one variable region of each of the heavy and light chains of an antibody. The Fab is the antigen determinant region of the antibody and can be generated by enzymatically cleaving the Fc region from an antibody.

In some embodiments of the first aspect of the invention, the antigen-recognition domain is a single-chain variable fragment (scFv) that binds to a dysfunctional P2X₇ receptor. As would be understood in the art, a scFv is a fusion protein comprising two portions that may share homology with, or may be identical to, the variable-heavy (VH) and variable-light (VL) chains of an antibody, with the two portions connected together with a linker peptide. For example, the scFv may include VH and VL amino acid sequences that are derived from an antibody that recognises a dysfunctional P2X₇ receptor. In this context it will be appreciated that the term "derived from" is not a reference to the source of the polypeptides *per se,* but rather refers to the derivation of the amino acid sequence that constitute a portion of the antigen-binding region. Consequently, the term "derived from" includes synthetically, artificially or otherwise created polypeptides that share sequence identity to an antibody that binds to the dysfunctional P2X₇ receptor.

In some embodiments of the first aspect of the invention, the antigen-recognition domain is a multivalent scFv that binds to a dysfunctional P2X₇ receptor. In some embodiments, the multivalent scFv is a di-valent or tri-valent scFv.

In some embodiments of the first aspect of the invention, the antigen-recognition domain is a single-antibody domain (sdAb) that binds to a dysfunctional P2X₇ receptor.

In some embodiments, the antigen-recognition domain includes the amino acid sequence set forth in SEQ ID NO: 10, SEQ ID NO: 32, SEQ ID NO: 33 or SEQ ID NO: 34 or a functional variant thereof.

In some embodiments, the antigen-recognition domain includes a binding peptide that includes amino acid sequence homology with one or more CDR regions of an antibody that binds to a dysfunctional P2X₇ receptor. In some embodiments, the antigen recognition domain includes three CDRs sequences spanning positions 30 to 35, 50 to 67 or 98 to 108 of the sequences set forth in SEQ ID NOS: 10, 32, 33 or 34. The sequences interspacing the CDR regions of the antigen binding peptides set forth in SEQ ID NOS: 10, 32, 33 or 34 can be any suitable sequence that permits the appropriate formation and conformation of the CDR regions. In some embodiments, the antigen recognition domain includes three CDR sequences set forth in SEQ ID NOS: 10, 32, 33 or 34.

Antibodies directed against dysfunctional P2X₇ receptors, from which suitable amino acid sequences may be derived, and methods for producing such antibodies, have been described in the art (for example WO2001/020155, WO2003/020762, WO2008/043145, WO2008/043146, WO2009/033233, WO2011/020155 and WO2011/075789). Methods for generating polyclonal and monoclonal antibodies for specific epitopes (such as those set forth previously) would be known to a person skilled in the art. By way of summary, a desired epitope (such as a segment of the dysfunctional P2X₇ receptor including the proline at position 210) is injected into a suitable host animal in the presence of an appropriate immunogenic carrier protein and an adjuvant. Serum is then collected from the immunized animal and the antibody can be isolated based on its antibody class or its antigen specificity. Following assessment of the suitability and specificity of the purified antibody, the antibody can be further processed to isolate antigen-binding fragments, or sequenced to identify the relevant VH and VL domains. Suitable epitopes for the production of antibodies directed against the dysfunctional P2X₇ receptor are known in the art (see WO2008/043146, WO2010/000041 and WO2009/033233 as examples).

The signalling domain of the CAR can be any suitable domain that is capable of inducing, or participating in the induction of, an intracellular signalling cascade upon activation of the CAR as a result of recognition of an antigen by the antigen-recognition domain of the CAR. The signalling domain of a CAR will be specifically chosen depending on the cellular outcome desired following activation of the CAR. Whilst there are many possible signalling domains, when used in immunotherapy and cancer therapy the signalling domains can be grouped into two general categories based on the receptor from which they are derived, namely activation receptors and co-stimulatory receptors (see further details below). Therefore, in some embodiments of the first aspect of the invention, the signalling domain includes a portion derived from an activation receptor. In some embodiments, the signalling domain includes a portion derived from a co-stimulatory receptor

As used throughout the specification the term "portion", when used with respect to an activation receptor or co-stimulatory receptor, relates to any segment of the receptor that includes a sequence responsible for, or involved in, the initiation/induction of an intracellular signalling cascade following interaction of the receptor with its cognate antigen or ligand. An example of the initiation/induction of an intracellular signalling cascade for the T cell receptor (TCR) via CD3 is outlined below.

Whilst not wishing to be bound by theory, the extracellular portion of the TCR largely comprises heterodimers of either the clonotypic TCRα and TCRβ chains (the TCRα/β receptor) or the TCRγ and TCRδ chains (the TCRγδ receptor). These TCR heterodimers generally lack inherent signalling transduction capabilities and therefore they are non-covalently associated with multiple signal transducing subunits of CD3 (primarily CD3-zeta, -gamma, -delta, and - epsilon). Each of the gamma, delta, and epsilon chains of CD3 has an intracellular (cytoplasmic) portion that includes a single Immune-receptor-Tyrosine-based-Activation-Motif (ITAM), whilst the CD3-zeta chain includes three tandem ITAMs. Upon engagement of the TCR by its cognate antigen in the presence of MHC, and the association of a requisite co-receptor such as CD4 or CD8, signalling is initiated which results in a tyrosine kinase (namely Lck) phosphorylating the two tyrosine residues within the intracellular ITAM(s) of the CD3 chains. Subsequently, a second tyrosine kinase (ZAP-70 - itself activated by Lck phosphorylation) is recruited to biphosphorylate the ITAMs. As a result, several downstream target proteins are activated which eventually leads to intracellular conformational changes, calcium mobilisation, and actin cytoskeleton re-arrangement that when combined ultimately lead to activation of transcription factors and induction of a T cell immune response.

As used throughout the specification the term "activation receptor" relates to receptors, or co-receptors that form a component of, or are involved in the formation of, the T cell receptor (TCR) complex, or receptors involved in the specific activation of immune cells as a result of recognition of an antigenic or other immunogenic stimuli.

Non-limiting examples of such activation receptors include components of the T cell receptor-CD3 complex (CD3-zeta, -gamma, -delta, and -epsilon), the CD4 co-receptor, the CD8 co-receptor, FC receptors or Natural Killer (NK) cell associated activation receptors such a LY-49 (KLRA1), natural cytotoxicity receptors (NCR, preferably NKp46, NKp44, NKp30 or NKG2 or the CD94/NKG2 heterodimer). Consequently, in some embodiments of the first aspect of the present invention, the signalling domain includes a portion derived from any one or more of a member of the CD3 co-receptor complex (preferably the CD3-ζ chain or a portion thereof), the CD4 co-receptor, the CD8 co-receptor, a Fc Receptor (FcR) (preferably the FcεRI or FcyRI) or NK associated receptors such a LY-49.

The specific intracellular signal transduction portion of each of the CD3 chains are known in the art. By way of example, the intracellular cytoplasmic region of the CD3ζ chain spans from amino acid 52 to amino acid 164 of the sequence set forth in SEQ ID NO: 4, with the three ITAM regions spanning amino acids 61 to 89, 100 to 128 and 131 to 159 of SEQ ID NO: 4. Furthermore, the intracellular portion of the CD3ε chain spans amino acids 153 to 207 of the sequence set forth in SEQ ID NO: 5, with the single ITAM region spanning amino acids 178 to 205 of SEQ ID NO: 5. The intracellular portion of CD3γ chain spans amino acids 138 to 182 of the sequence set forth in SEQ ID NO: 6 with the single ITAM region spanning amino acids amino acids 149 to 177 of SEQ ID NO: 6. The intracellular portion of CD3δ spans amino acids 127 to 171 of the sequence set forth in SEQ ID NO: 7 with the single ITAM region spanning amino acids 138 to 166 of SEQ ID NO: 7.

In some embodiments of the first aspect of the present invention, the signalling domain includes a portion derived from any one of CD3 (preferably the CD3-ζ chain or a portion thereof) or an FC receptor (preferably the FcεRI or FcyRI). In some embodiments, the portion of the CD3-ζ co-receptor complex includes the amino acid sequence set forth in SEQ ID NO: 22, or a functional variant thereof.

The intracellular portions of the FC receptors are known in the art. For example, the intracellular portions of the FcεR1 span amino acids 1 to 59, 118 to 130 and 201 to 244 of the sequence set forth in SEQ ID NO: 8. Furthermore, the intracellular portion of FcγRI spans the amino acids 314 to 374 of the sequence set forth in SEQ ID NO: 9.

Various combinations of portions of activation receptors can be utilized to form the transmembrane (TM) and intracellular (IC) portions of the CAR for example the CD3ζ TM and CD3ζ IC (Landmeier S. et al. Cancer Res. 2007; 67:8335-43; Guest RD. et al., J Immunother. 2005, 28:203-11; Hombach AA. et al. J Immunol. 2007; 178: 4650-7), the CD4 TM and CD3ζ IC (James SE. et al. J Immunol. 2008;180:7028-38), the CD8 TM and CD3ζ IC (Patel SD. et al. Gene Ther. 1999; 6: 412-9), and the FcεRIγ TM and the FcεRIγ IC (Haynes NM. et al. J Immunol. 2001; 166: 182-7; Annenkov AE. et al. J Immunol. 1998; 161: 6604-13).

As used throughout the specification the term "co-stimulatory receptor" relates to receptors or co-receptors that assist in the activation of an immune cell upon antigen specific inducement of an activation receptor. As will be understood, co-stimulatory receptors do not require the presence of antigen and are not antigen specific, but are typically one of two signals, the other being an activation signal, which is required for the induction of an immune cellular response. In the context of an immune response a co-stimulation receptor is typically activated by the presence of its expressed ligand on the surface of an antigen-presenting cell (APC) such as a dendritic cell or macrophage. With specific regard to T cells, co-stimulation is necessary to lead to cellular activation, proliferation, differentiation and survival (all of which are generally referred to under the umbrella of T cell activation), whilst presentation of an antigen to a T cell in the absence of co-stimulation can lead to anergy, clonal deletion and/or the development of antigen specific tolerance. Importantly, co-stimulatory molecules can inform the T cell response to a simultaneously encountered antigen. Generally, an antigen encountered in the context of a 'positive' co-stimulatory molecule will lead to activation of the T cell and a cellular immune response aimed at eliminating cells expressing that antigen. Whilst an antigen encountered in the context of a 'negative' co-receptor will lead to an induced state of tolerance to the co-encountered antigen.

Non-limiting examples of T cell co-stimulatory receptors include CD27, CD28, CD30, CD40, DAP10, OX40, 4-1BB (CD137), ICOS. Specifically, CD27, CD28, CD30, CD40, DAP10, OX40, 4-1BB (CD137), and ICOS all represent 'positive' co-stimulatory molecules that enhance activation of a T cell response. Accordingly, in some embodiments of the first aspect of the present invention, the signalling domain includes a portion derived from any one or more of CD27, CD28, CD30, CD40, DAP10, OX40, 4-1BB (CD137) and ICOS.

In some embodiments of the first aspect of the present invention, the signalling domain includes a portion derived from the CD28, OX40 or 4-1BB co-stimulatory receptors. In some embodiments, the signalling domain includes a portion of the CD28 co-stimulatory receptor. In some embodiments, the signalling domain includes a portion of the OX40 co-stimulatory receptor. In some embodiments, the portion of the OX40 co-stimulatory receptor includes the amino acid sequence set forth in SEQ ID NO: 20, or a functional variant thereof.

Various combinations of portions of co-stimulatory receptors can be utilized to form the transmembrane (TM) and intracellular (IC) portions of the CAR. For example the CD8 TM and DAP10 IC or CD8 TM and 4-1BB IC (Marin V. et al. Exp Hematol. 2007; 35: 1388-97), the CD28 TM and the CD28 IC (Wilkie S. et al. J Immunol. 2008;180: 4901-9; Maher J. et al. Nat Biotechnol. 2002; 20: 70-5), and the CD8 TM and the CD28 IC (Marin V. et al. Exp Hematol. 2007; 35: 1388-97).

Sequence information for the above-referenced activation and co-stimulatory receptors is readily accessible in a variety of databases. For example, embodiments of human amino acid, gene and mRNA sequences for these receptors is provided in Table 2.

**TABLE 2**

| **Summary of Activation and Co-stimulation Receptor Sequence Information** | | | |
|---|---|---|---|
| **Receptor Name** | **Uniprot Ref No.** | **NCBI Gene ID No.** | **GeneBank mRNA Ref No.** |
| CD3-zeta | P20963 | 919 | GI:166362721 |
| CD3-gamma | P09693 | 917 | GI:166362738 |
| CD3-delta | P04234 | 915 | GI:98985799 |
| CD3-epsilon | P07766 | 916 | GI:166362733 |
| CD4 | P0173 | 920 | GI:303522473 |
| CD8 alpha | P01732 | 925 | GI:225007534 |
| CD8 beta | P01966 | 926 | GI:296010927 |
| FcγRI | P12314 | 2209 | GI:31331 |
| FcεR1 | Q01362 | 2206 | GI:219881 |
| Ly-49 (KLRAI) | Q 7Z556 | 10748 | GI:33114184 |
| NKp46 | O76036 | 9437 | GI:3647268 |
| NKp44 | O95944 | 9436 | GI:4493701 |
| NKp30 | O14931 | 259197 | GI:5823969 |
| CD94 | Q13241 | 3824 | GI:1098616 |
| CD27 | P26842 | 939 | GI:180084 |
| CD28 | P10747 | 940 | GI:338444 |
| CD30 | P28908 | 943 | GI:180095 |
| CD40 | P25942 | 958 | GI:29850 |
| DAP10 | Q9UBK5 | 10870 | GI:5738198 |
| OX40 | P43489 | 7293 | GI:472957 |
| 4-1BB (CD137) | Q07011 | 3604 | GI:571320 |
| ICOS | Q9Y6W8 | 29851 | GI:9968295 |
| CTLA-4 | P16410 | 1493 | GI:291928 |
| PD-1 | Q15116 | 5133 | GI:2149002 |

Whilst Table 2 is provided with reference to human activation and co-stimulatory receptors, it would be understood by a person skilled in the art that homologous and orthologous versions of each receptor are present in the majority of mammalian and vertebrate species. Therefore, the above-referenced sequences are only provided as non-limiting examples of receptor sequences that may be included in a CAR of the first aspect of the present invention and homologous and orthologous sequences from any desired species may be used to generate a CAR that is suitable for the given species.

In some embodiments of the first aspect of the invention, the signalling domain includes a portion derived from an activation receptor and a portion derived from a co-stimulatory receptor. Whilst not wishing to be bound by theory, in this context the recognition of an antigen by the antigen-recognition domain of the CAR will simultaneously induce both an intracellular activation signal and an intracellular co-stimulatory signal. Consequently, this will simulate the presentation of an antigen by an APC expressing co-stimulatory ligand. Alternatively, the CAR could have a signalling domain that includes a portion derived from either an activation receptor or a co-stimulatory receptor. In this alternative form, the CAR will only induce either an activating intracellular signalling cascade or a co-stimulatory intracellular signalling cascade.

In some embodiments of the first aspect of the invention, the CAR will have a signalling domain that includes a portion derived from a single activation receptor and portions derived from multiple co-stimulatory receptors. In some embodiments, the CAR will have a signalling domain that includes portions derived from multiple activation receptors and a portion derived from a single co-stimulatory receptor. In some embodiments, the CAR will have a signalling domain that includes portions derived from multiple activation receptors and portions derived from multiple co-stimulatory receptors. In some embodiments, the CAR will have a signalling domain that includes a portion derived from a single activation receptor and portions derived from two co-stimulatory receptors. In some embodiments, the CAR will have a signalling domain that includes a portion derived from a single activation receptor and portions derived from three co-stimulatory receptors. In some embodiments, the CAR will have a signalling domain that includes portions derived from two activation receptors, and a portion derived from one co-stimulatory receptor. In some embodiments, the CAR will have a signalling domain that includes portions derived from two activation receptors and portions derived from two co-stimulatory receptors. As will be understood there are further variations of the number of activation receptors and co-stimulatory receptors from which the signalling domain can be derived from, and the above examples are not considered to be limiting on the possible combinations included herein.

In some embodiments of the first aspect of the invention, the chimeric antigen receptor includes the amino acid sequence set forth in SEQ ID NO: 26,SEQ ID NO: 27, SEQ ID NO: 52, SEQ ID NO: 53 or SEQ ID NO: 54.

As indicated above, the present invention includes a functional variant of any one of SEQ ID NO: 10, SEQ ID NO: 18, SEQ ID NO: 20, SEQ ID NO: 22, SEQ ID NO: 26, SEQ ID NO: 27, SEQ ID NO: 32, SEQ ID NO: 33, SEQ ID NO: 34, SEQ ID NO: 52, SEQ ID NO: 53 or SEQ ID NO: 54. In the context of the present invention, a "functional variant" may include any amino acid sequence provided it maintains the function of any one of SEQ ID NO: 10, SEQ ID NO: 18, SEQ ID NO: 20, SEQ ID NO: 22, SEQ ID NO: 26, SEQ ID NO: 27, SEQ ID NO: 32, SEQ ID NO: 33, SEQ ID NO: 34, SEQ ID NO: 52, SEQ ID NO: 53 or SEQ ID NO: 54.

As such, the functional variant may, for example, have one or more amino acid insertions, deletions or substitutions relative to one of SEQ ID NO: 10, SEQ ID NO: 18, SEQ ID NO: 20, SEQ ID NO: 22, SEQ ID NO: 26, SEQ ID NO: 27, SEQ ID NO: 32, SEQ ID NO: 33, SEQ ID NO: 34, SEQ ID NO: 52, SEQ ID NO: 53 or SEQ ID NO: 54; a mutant form or allelic variant of one of SEQ ID NO: 10, SEQ ID NO: 18, SEQ ID NO: 20, SEQ ID NO: 22, SEQ ID NO: 26, SEQ ID NO: 27, SEQ ID NO: 32, SEQ ID NO: 33, SEQ ID NO: 34, SEQ ID NO: 52, SEQ ID NO: 53 or SEQ ID NO: 54; an ortholog of one of SEQ ID NO: 10, SEQ ID NO: 18, SEQ ID NO: 20, SEQ ID NO: 22, SEQ ID NO: 26, SEQ ID NO: 27, SEQ ID NO: 32, SEQ ID NO: 33, SEQ ID NO: 34, SEQ ID NO: 52, SEQ ID NO: 53 or SEQ ID NO: 54; a homeologue of one of SEQ ID NO: 10, SEQ ID NO: 18, SEQ ID NO: 20, SEQ ID NO: 22, SEQ ID NO: 26, SEQ ID NO: 27, SEQ ID NO: 32, SEQ ID NO: 33, SEQ ID NO: 34, SEQ ID NO: 52, SEQ ID NO: 53 or SEQ ID NO: 54; an analog of one of SEQ ID NO: 10, SEQ ID NO: 18, SEQ ID NO: 20, SEQ ID NO: 22, SEQ ID NO: 26,SEQ ID NO: 27, SEQ ID NO: 32, SEQ ID NO: 33, SEQ ID NO: 34, SEQ ID NO: 52, SEQ ID NO: 53 or SEQ ID NO: 54; and the like, provided the functional variant maintains the function of any one of SEQ ID NO: 10, SEQ ID NO: 18, SEQ ID NO: 20, SEQ ID NO: 22, SEQ ID NO: 26, SEQ ID NO: 27, SEQ ID NO: 32, SEQ ID NO: 33, SEQ ID NO: 34, SEQ ID NO: 52, SEQ ID NO: 53 or SEQ ID NO: 54.

For example with respect to SEQ ID NO: 26, SEQ ID NO: 27, SEQ ID NO: 52, SEQ ID NO: 53 or SEQ ID NO: 54 the function of a chimeric antigen receptor comprising SEQ ID NO: 26, SEQ ID NO: 27, SEQ ID NO: 52, SEQ ID NO: 53 or SEQ ID NO: 54 is to recognise a dysfunctional P2X₇ receptor without significant recognition of the functional P2X₇ receptor, and induce an intracellular signal which results in the activation of a T cell expressing the CAR. As would be understood by a person skilled in the art, variation to portions of the amino acid sequence of the chimeric antigen receptor set forth in SEQ ID NO: 26, SEQ ID NO: 27, SEQ ID NO: 52, SEQ ID NO: 53 or SEQ ID NO: 54 may be made without significant alteration of the recognition of the dysfunctional P2X₇ receptor and/or activation of a T cell expressing the CAR. Such variations may include, but are not limited to, variations in the hinge region of the chimeric antigen receptor, variations in the transmembrane domain, and variations in the portions of the activation receptors and/or co-stimulatory receptors that comprise the intracellular domain of the chimeric antigen receptor.

As indicated above, the functional variant may comprise individual amino acid substitutions, deletions or insertions relative to one of SEQ ID NO: 10, SEQ ID NO: 18, SEQ ID NO: 20, SEQ ID NO: 22, SEQ ID NO: 26, SEQ ID NO: 27, SEQ ID NO: 32, SEQ ID NO: 33, SEQ ID NO: 34, SEQ ID NO: 52, SEQ ID NO: 53 or SEQ ID NO: 54. For example, a person skilled in the art will recognise that any amino acid can be substituted with a chemically (functionally) similar amino acid and retain function of the polypeptide. Such conservative amino acid substitutions are well known in the art. The following groups in Table 3 each contain amino acids that are conservative substitutions for one another.

**TABLE 3**

| **Exemplary amino acid conservative substitutions** | | | |
|---|---|---|---|
| **Original Residue** | **Exemplary Substitutions** | **Original Residue** | **Exemplary Substitutions** |
| Ala (A) | Val (V), Leu (L), Ile (I), Gly (G) | Leu (L) | Ile (I), Val (V), Met (M), Ala (A), Phe (F) |
| Arg (R) | I vs (K) | Lys (K) | Arg (R) |
| Asn (N) | Gln (Q), His (H) | Met (M) | Leu (L), Phe (F) |
| Asp (D) | Glu (E) | Phe (F) | Leu (L), Val (V), AlaI (A) |
| Cys (C) | Ser (S) | Pro (P) | Gly (G) |
| Gln (Q) | Asn (N), His (H) | Ser (S) | Thr (T) |
| Glu (E) | Asp (D) | Thr (T) | Ser (S) |
| Gly (G) | Pro (P), Ala (A) | Trp (W) | Tyr (Y) |
| His (H) | Asn (N), Gln (Q) | Tyr (Y) | Trp (W), Phe (F) |
| Ile (I) | Leu (L), Val (V), Ala (A) | Val (V) | Ile (I), Leu (L), Met (M), Phe (F), Ala (A) |

Furthermore, if desired, unnatural amino acids or chemical amino acid analogues can be introduced as a substitution or addition into a polypeptide encompassed herein. Such amino acids include, but are not limited to, the D-isomers of the common amino acids, 2,4-diaminobutyric acid, α-amino isobutyric acid, 4-aminobutyric acid, 2-aminobutyric acid, 6-amino hexanoic acid, 2-amino isobutyric acid, 3-amino propionic acid, ornithine, norleucine, norvaline, hydroxyproline, sarcosine, citrulline, homocitrulline, cysteic acid, t-butylglycine, t-butylalanine, phenylglycine, cyclohexylalanine, β-alanine, fluoro-amino acids, designer amino acids such as β-methyl amino acids, Cα-methyl amino acids, Nα-methyl amino acids, and amino acid analogues in general.

As set out above, a functional variant of any one of SEQ ID NO: 10, SEQ ID NO: 18, SEQ ID NO: 20, SEQ ID NO: 22, SEQ ID NO: 26, SEQ ID NO: 27, SEQ ID NO: 32, SEQ ID NO: 33, SEQ ID NO: 34, SEQ ID NO: 52, SEQ ID NO: 53 or SEQ ID NO: 54 may comprise an amino acid sequence which is at least 80% identical to any one of SEQ ID NO: 10, SEQ ID NO: 18, SEQ ID NO: 20, SEQ ID NO: 22, SEQ ID NO: 26, SEQ ID NO: 27, SEQ ID NO: 32, SEQ ID NO: 33, SEQ ID NO: 34, SEQ ID NO: 52, SEQ ID NO: 53 or SEQ ID NO: 54. In other embodiments, a functional variant may comprise at least 85% amino acid sequence identity, at least 90% amino acid sequence identity, at least 91% amino acid sequence identity, at least 92% amino acid sequence identity, at least 93% amino acid sequence identity, at least 94% amino acid sequence identity, at least 95% amino acid sequence identity, at least 96% amino acid sequence identity, at least 97% amino acid sequence identity, at least 98% amino acid sequence identity, at least 99% amino acid sequence identity, or at least 99.1%, 99.2%, 99.3%, 99.4%, 99.5%, 99.6%, 99.7%, 99.8%, or 99.9% amino acid sequence identity to any one of SEQ ID NO: 10, SEQ ID NO: 18, SEQ ID NO: 20, SEQ ID NO: 22, SEQ ID NO: 26, SEQ ID NO: 27, SEQ ID NO: 32, SEQ ID NO: 33, SEQ ID NO: 34, SEQ ID NO: 52, SEQ ID NO: 53 or SEQ ID NO: 54.

When comparing amino acid sequences, the sequences should be compared over a comparison window which is determined by the length of the polypeptide. For example, a comparison window of at least 20 amino acid residues, at least 50 amino acid residues, at least 75 amino acid residues, at least 100 amino acid residues, at least 200 amino acid residues, at least 300 amino acid residues, at least 400 amino acid residues, at least 500 amino acid residues, at least 600 amino acid residues, or over the full length of any one of SEQ ID NO: 10, SEQ ID NO: 18, SEQ ID NO: 20, SEQ ID NO: 22, SEQ ID NO: 26, SEQ ID NO: 27, SEQ ID NO: 32, SEQ ID NO: 33, SEQ ID NO: 34, SEQ ID NO: 52, SEQ ID NO: 53 or SEQ ID NO: 54 is envisaged. The comparison window may comprise additions or deletions (i.e. gaps) of about 20% or less as compared to the reference sequence (which does not comprise additions or deletions) for optimal alignment of the two sequences. Optimal alignment of sequences for aligning a comparison window may be conducted by computerised implementations of algorithms such as the BLAST family of programs as, for example, disclosed by Altschul et al., 1997, Nucl. Acids Res. 25: 3389-3402. Global alignment programs may also be used to align similar sequences of roughly equal size. Examples of global alignment programs include NEEDLE (available at www.ebi.ac.uk/Tools/psa/emboss_needle/) which is part of the EMBOSS package (Rice P et al., 2000, Trends Genet., 16: 276-277), and the GGSEARCH program (available at fasta.bioch.virginia.edu/fasta_www2/fasta_www.cgi?rm=compare&pgm=gnw) which is part of the FASTA package (Pearson W and Lipman D, 1988, Proc. Natl. Acad. Sci. USA, 85: 2444-2448). Both of these programs are based on the Needleman-Wunsch algorithm which is used to find the optimum alignment (including gaps) of two sequences along their entire length. A detailed discussion of sequence analysis can also be found in Unit 19.3 of Ausubel et al ("Current Protocols in Molecular Biology" John Wiley & Sons Inc, 1994-1998, Chapter 15, 1998).

In a second aspect, the present invention provides a nucleic acid molecule including a nucleotide sequence encoding the chimeric antigen receptor according to the first aspect of the invention. In some embodiments, the nucleic acid molecule is a non-naturally occurring nucleic acid molecule.

In some embodiments of the second aspect of the invention, the nucleic acid molecule includes a nucleotide sequence which encodes the amino acid sequence set forth in SEQ ID NO: 26, SEQ ID NO: 27, SEQ ID NO: 52, SEQ ID NO: 53 or SEQ ID NO: 54, or encodes a functional variant of SEQ ID NO: 26, SEQ ID NO: 27, SEQ ID NO: 52, SEQ ID NO: 53 or SEQ ID NO: 54. In some embodiments, the functional variant includes an amino acid sequence which is at least 80% identical to SEQ ID NO: 26, SEQ ID NO: 27, SEQ ID NO: 52, SEQ ID NO: 53 or SEQ ID NO: 54.

The nucleic acid molecule may comprise any polyribonucleotide or polydeoxyribonucleotide, which may be unmodified, or modified, RNA or DNA. For example, the nucleic acid molecule may include single- and/or double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single-and double-stranded regions. In addition, the nucleic acid molecule may comprise triple-stranded regions comprising RNA or DNA or both RNA and DNA. The nucleic acid molecule may also comprise one or more modified bases or DNA or RNA backbones modified for stability or for other reasons. A variety of modifications can be made to DNA and RNA; thus the term "nucleic acid molecule" embraces chemically, enzymatically, or metabolically modified forms.

In some embodiments of the second aspect of the invention, the nucleic acid molecule includes the nucleotide sequence set forth in SEQ ID NO: 28, SEQ ID NO: 29, SEQ ID NO: 35, SEQ ID NO: 36 or SEQ ID NO: 37.

It would be understood by a person skilled in the art that any nucleotide sequence which encodes a chimeric antigen receptor having the amino acid sequence set forth in SEQ ID NO: 26, SEQ ID NO: 27, SEQ ID NO: 35, SEQ ID NO: 36 or SEQ ID NO: 37, or a functional variant of SEQ ID NO: 26, SEQ ID NO: 27, SEQ ID NO: 35, SEQ ID NO: 36 or SEQ ID NO: 37, is contemplated by the present invention. For example, variants of SEQ ID NO: 28, SEQ ID NO: 29, SEQ ID NO: 35, SEQ ID NO: 36 or SEQ ID NO: 37 are contemplated which comprise one or more different nucleic acids to SEQ ID NO: 28, SEQ ID NO: 29, SEQ ID NO: 35, SEQ ID NO: 36 or SEQ ID NO: 37 but which still encode identical amino acid sequences. Because of the degeneracy of the genetic code, a large number of nucleic acids can encode any given protein. For instance, the codons GCA, GCC, GCG and GCU all encode the amino acid alanine. Therefore, at every position in SEQ ID NO: 28, SEQ ID NO: 29, SEQ ID NO: 35, SEQ ID NO: 36 or SEQ ID NO: 37 where an alanine is specified by a codon, the codon can be altered to any of the corresponding codons described without altering the encoded polypeptide. Accordingly, every nucleotide sequence herein which encodes a chimeric antigen receptor having the amino acid sequence set forth in SEQ ID NO: 26, SEQ ID NO: 27, SEQ ID NO: 35, SEQ ID NO: 36 or SEQ ID NO: 37, or a functional variant of SEQ ID NO: 26, SEQ ID NO: 27, SEQ ID NO: 35, SEQ ID NO: 36 or SEQ ID NO: 37 also describes every possible silent variation of the nucleotide sequence. One of skill will recognise that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleotide sequence that encodes a polypeptide is implicit in each described sequence.

In a third aspect, the present invention provides a viral vector including a nucleic acid according to the second aspect.In one embodiment, the method for introducing the nucleic acid construct into a host cell is viral transduction. Methods are known in the art for utilising viral transduction to elicit expression of a CAR in a PBMC (Parker, LL. et al. Hum Gene Ther. 2000;11: 2377-87) and more generally utilising retroviral systems for transduction of mammalian cells (Cepko, C. and Pear, W. Curr Protoc Mol Biol. 2001, unit 9.9). In other embodiments, the nucleic acid construct is a plasmid, a cosmid, an artificial chromosome or the like, and can be transfected into the cell by any suitable method known in the art.

In a fourth aspect the present invention provides a genetically modified cell that includes the chimeric antigen receptor according to the first aspect of the invention.

In some embodiments of the fourth aspect of the invention, the genetically modified cell includes two or more different CARs.

In a fifth aspect the invention provides a genetically modified cell that includes the nucleic acid molecule according to the second aspect of the invention, or includes the nucleic acid construct according to the third aspect of the invention, or a genomically integrated form of the nucleic acid construct.

In some embodiments of the fifth aspect of the invention, the genetically modified cell includes a nucleic acid molecule, or a nucleic acid construct, that encodes for two or more different CARs. In some embodiments of the fifth aspect of the invention, the genetically modified cell includes two or more nucleic acid molecules, or two or more nucleic acid constructs, each of which encodes for a different CAR.

As referred to herein, a "genetically modified cell" includes any cell comprising a non-naturally occurring and/or introduced nucleic acid molecule or nucleic acid construct encompassed by the present invention. The introduced nucleic acid molecule or nucleic acid construct may be maintained in the cell as a discreet DNA molecule, or it may be integrated into the genomic DNA of the cell.

Genomic DNA of a cell should be understood in its broadest context to include any and all endogenous DNA that makes up the genetic complement of a cell. As such, the genomic DNA of a cell should be understood to include chromosomes, mitochondrial DNA and the like. As such, the term "genomically integrated" contemplates chromosomal integration, mitochondrial DNA integration, and the like. The "genomically integrated form" of the construct may be all or part of the construct. However, in some embodiments the genomically integrated form of the construct at least includes the nucleic acid molecule of the second aspect of the invention.

As used herein, the term "different CARs" or "different chimeric antigen receptors" refers to any two or more CARs that have either non-identical antigen-recognition and/or non-identical signalling domains. In one example, "different CARs" includes two CARs with the same antigen-recognition domains (e.g. both CARs may recognise a dysfunctional P2X₇ receptor), but have different signalling domains, such as one CAR having a signalling domain with a portion of an activation receptor and the other CAR having a signalling domain with a portion of an co-stimulatory receptor. As will be understood, at least one of the two or more CARs within this embodiment will have an antigen-recognition domain that recognises the dysfunctional P2X₇ receptor and the other CAR(s) may take any suitable form and may be directed against any suitable antigen.

Accordingly, in some embodiments of the fourth and fifth aspects of the invention the two or more different CARs have different signalling domains, and may have identical, or different, antigen-recognition domains. Specifically, the genetically modified cell according to the fourth or fifth aspects of the invention may include a first chimeric antigen receptor with a signalling domain that includes a portion derived from an activation receptor and a second chimeric antigen receptor with a signalling domain including a portion derived from a co-stimulatory receptor.

In some embodiments of the fourth or fifth aspects of the invention, the activation receptor (from which a portion of signalling domain is derived) is the CD3 co-receptor complex or is an Fc receptor.

In some embodiments of the fourth or fifth aspects of the invention, the co-stimulatory receptor (from which a portion of signalling domain is derived) is selected from the group consisting of CD27, CD28, CD-30, CD40, DAP10, OX40, 4-1BB (CD137) and ICOS.

In some embodiments of the fourth or fifth aspects of the invention, the co-stimulatory receptor (from which a portion of signalling domain is derived) is selected from the group consisting of CD28, OX40 or 4-1BB.

In some embodiments of the fourth and fifth aspects of the invention, the genetically modified cell is further modified to constitutively express co-stimulatory receptors.

As described above, a cellular immune response is typically only induced when an activation signal (typically in response to an antigen) and a co-stimulation signal are simultaneously experienced. Therefore, by having a genetically modified cell in accordance with some of the above embodiments, which includes two or more CARs that in combination provide both an intracellular activation signal and an intracellular co-stimulation signal, ensures that a sufficient immune response can be induce in response to the recognition by the CAR(s) of their cognate antigen. Alternatively, the genetically modified cell may include only one CAR, which has an antigen-recognition domain that recognises a dysfunctional P2X₇ receptor, and may constitutively express co-stimulatory receptors, thereby increasing the likelihood of co-stimulation being provided simultaneously when the CAR is activated. Alternatively, the genetically modified cell may be further modified to constitutively express both co-stimulatory receptor(s) and its/their ligand(s). In this way the cell is continuously experiencing co-stimulation and only needs the activation of a CAR, with a signalling domain including a portion from an activation receptor, for immune activation of the cell.

Therefore in some embodiments of the fourth or fifth aspects of the invention, the genetically modified cell is further modified so as to constitutively express co-stimulatory receptors. In further embodiments, the genetically modified cell is further modified so as to express ligands for the co-stimulatory receptors, thereby facilitating auto-stimulation of the cell. Examples of CAR-expressing T cells that also express both co-stimulatory receptors and their cognate ligands (so as to induce auto-stimulation) are known in the art and include, *inter alia,* those disclosed in Stephen MT. et al. Nat Med, 2007; 13: 1440-9.

The potency of a genetically modified cell including a CAR can be enhanced by further modifying the cell so as to secrete cytokines, preferably proinflammatory or proproliferative cytokines. This secretion of cytokines provide both autocrine support for the cell expressing the CAR, and alters the local environment surrounding the CAR-expressing cell such that other cells of the immune system are recruited and activated. Consequently, in some embodiments of the fourth or fifth aspects of the invention the genetically modified cell is further modified to secret cytokines. This secretion may be constitutive, or may be inducible upon recognition of a CAR of its cognate antigen of ligand.

Whilst any one or more cytokines can be selected depending on the desired immune response, preferable cytokines include IL-2, IL-7, IL-12, IL-15, IL-17 and IL-21, or a combination thereof.

The genetically modified cell of the fourth or fifth aspects of the invention can be any suitable immune cell, or can be a homogeneous or a heterogeneous cell population. In some embodiments, the cell is a leukocyte, a Peripheral Blood Mononuclear Cell (PBMC), a lymphocyte, a T cell, a CD4+ T cell, a CD8+ T cell, a natural killer cell or a natural killer T cell.

In a sixth aspect, the present invention provides a genetically modified cell of the fourth or fifth aspect of the invention of use in a method of killing a cell expressing a dysfunctional P2X₇ receptor, the method including exposing the cell expressing a dysfunctional P2X₇ receptor to the genetically modified cell.

Therefore, in some embodiments of the sixth aspect of the invention, the CAR directly recognises the dysfunctional P2X₇ receptor. In other embodiments, the CAR indirectly recognises the dysfunctional P2X₇ receptor.

As used herein the term "directly recognises" includes direct binding of the antigen-recognition domain of the CAR to the dysfunctional P2X₇ receptor, or an epitope thereof, when the receptor is present in its natural form. In another non-limiting example, the antigen-recognition domain may directly bind to a processed form of the dysfunctional P2X₇ receptor, which may be presented by antigen presenting molecules such as the major histocompatibility complex (MHC).

As an alternative to the CAR directly recognising a cell having a dysfunctional P2X₇ receptor, the CAR may be directed against a cell having a dysfunctional P2X₇ receptor by an indirect means.

Consequently, in some embodiments of the sixth aspect of the invention, the chimeric antigen receptor recognises the dysfunctional P2X₇ receptor via an intermediate. An intermediate may be a molecule such as a probe that binds or interacts directly with the dysfunctional P2X₇ receptor. Non-limiting examples of such probes include antibodies, a Fab of an antibody, a scFv, a soluble engineered TCR or an aptamer. The CAR may be able to directly recognise the probe or the probe may have a tag that is recognised by the CAR. In either regard the probe provides the specificity for the target cell (namely a cell having a dysfunctional P2X₇ receptor) whilst the genetically modified cell having the CAR provides the efficacy and directs an immune response against the target cell. Alternatively, the intermediate could be a cell endogenous marker which is associated with, or its expression is correlated to, the dysfunctional P2X₇ receptor. The dysregulation of the marker may be a cause of or a result of the dysfunction of the P2X₇ receptor.

In some embodiments of the sixth aspect of the invention, the method of killing a cell having a dysfunctional P2X₇ receptor further includes the step of exposing the cell having a dysfunctional P2X₇ receptor to an intermediate.

In some embodiments of the sixth aspect of the invention, the intermediate is a probe that binds to a dysfunctional P2X₇ receptor and the chimeric antigen receptor recognises the probe. Preferably the probe is an antibody or an aptamer.

The term "aptamer" as used throughout the specification refers to any oligonucleic acid, polynucleic acid, peptide or polypeptide which specifically binds to, or preferentially forms a complex with, a target (specifically a dysfunctional P2X₇ receptor).

In some embodiments of the sixth aspect of the invention, the probe includes a tag and the chimeric antigen receptor recognises the tag. Examples of a CAR that recognise cells by way of an intermediate are known in the art, for example European patent application EP 2651442.

In some embodiments of the sixth aspect of the invention, the cell having a dysfunctional P2X₇ receptor is within the body of a subject. In some embodiments, the subject is a human. In some embodiments, the method further includes exposing the cell expressing a dysfunctional P2X₇ receptor to a genetically modified together with an exogenous cytokine.

In some embodiments of the sixth aspect of the invention, the genetically modified cell is a genetically modified cell autologous to the cell expressing a dysfunctional P2X₇ receptorfrom the subject.

In some embodiments of the sixth aspect of the invention, the cell expressing a dysfunctional P2X₇ receptor is within the body of a subject. In some embodiments of the sixth aspect of the invention, the cell expressing a dysfunctional P2X₇ receptor is a cancer cell.

In some embodiments of the sixth aspect, the present invention provides a method of treating or preventing cancer in a subject, the method including providing a subject with a genetically modified cell having a chimeric antigen receptor, wherein the chimeric antigen receptor is directed against a target cell having a dysfunctional P2X₇ receptor.

The terms "treat", "treating" or "treatment," as used herein are to be understood to include within their scope one or more of the following outcomes: (i) inhibiting to some extent the growth of a primary tumour in a subject, including, slowing down and complete growth arrest, and including reducing the growth of the primary tumour after resection; (ii) inhibiting to some extent the growth and formation of one or more secondary tumours in a subject; (iii) reducing the number of tumour cells in a subject; (iv) reducing the size of a tumour in the subject; (v) inhibiting (i.e. reduction, slowing down or complete stopping) of tumour cell infiltration into peripheral organs; (vi) inhibiting (i.e. reduction, slowing down or complete stopping) of metastasis; (vii) improving the life expectancy of a subject as compared to the untreated state; (viii) improving the quality of life of a subject as compared to the untreated state; (ix) alleviating, abating or ameliorating at least one symptom of cancer in a subject; (x) causing regression or remission of cancer in a subject; (xi) relieving a condition in a subject that is caused by cancer; and (xii) stopping symptoms in a subject that are associated with cancer.

The terms "prevent" or "preventing" as used herein are to be understood to include within their scope inhibiting the formation of a primary tumour in a subject, inhibiting the formation of one or more secondary tumours in a subject, or reducing or eliminating the recurrence of cancer in a subject in remission.

The term "inhibiting" as used herein is taken to mean a decrease or reduction in the growth of a cancer, cancerous cell or tumour when compared to the growth in a control, such as an untreated cell or subject. In some embodiments, growth may be decreased or reduced by at least 10%, 20%, 30%, 40%, 50%, 60%, 70%, 80%, 90%, or 100%, relative to an untreated control.

Inhibition of the growth of a cancer, tumour or cancerous cell may be assessed by a range of methods known in the art. For example, for a cancerous cell *in vitro,* the growth of the cell may be determined by a suitable proliferation assay, or by method which assess the extent of incorporation of tritiated thymidine into cellular DNA over a given period of time. For a tumour or cancerous cell present *in vivo,* the growth of the tumour or cell may be determined for example by a suitable imaging method known in the art.

The term "subject" as used herein refers to any animal capable of suffering from cancer. Particular subjects of interest are human beings, and scientifically relevant species such as mice, rats, ferrets, guinea pigs, hamsters, non-human primates, dogs, pigs and sheep, or economically relevant animals such as horses, dogs, cats and cattle. In a preferred embodiment of the sixth aspect of the invention, the subject is a human.

A reference to "providing a subject with" relates to administering to the subject the genetically modified cell. Alternatively, the genetically modified cell may be generated within the subject. For example, the genetically modified cell may be generated *in vivo* such that the subject has an endogenous population of genetically modified cells. Suitable means for such *in vivo* generation are known in the art and include gene therapy of a subject.

As used throughout the specification a reference to a CAR being "directed" against a target cell having a dysfunctional P2X₇ receptor contemplates the selective targeting of an immune response toward a cell based on the cell having a dysfunctional P2X₇ receptor. Importantly, such targeting is not limited to direct recognition of the dysfunctional P2X₇ receptor by a CAR. That is to say that the CAR itself does not have to directly recognise or bind to the dysfunctional P2X₇ receptor but merely has to be able to selectively recognise and be activated by a cell that expresses a dysfunctional P2X₇ receptor.

Therefore, in some embodiments of the sixth aspect of the invention, the CAR directly recognises the dysfunctional P2X₇ receptor. In other embodiments, the CAR indirectly recognises the dysfunctional P2X₇ receptor.

As used herein the term "directly recognises" includes direct binding of the antigen-recognition domain of the CAR to the dysfunctional P2X₇ receptor, or an epitope thereof, when the receptor is present in its natural form. In another non-limiting example, the antigen-recognition domain may directly bind to a processed form of the dysfunctional P2X₇ receptor, which may be presented by antigen presenting molecules such as the major histocompatibility complex (MHC).

As an alternative to the CAR directly recognising a cell having a dysfunctional P2X₇ receptor, the CAR may be directed against a target cell having a dysfunctional P2X₇ receptor by an indirect means.

Consequently, in some embodiments of the sixth aspect of the invention, the chimeric antigen receptor recognises the dysfunctional P2X₇ receptor via an intermediate. An intermediate may be a molecule such as a probe that binds or interacts directly with the dysfunctional P2X₇ receptor. Non-limiting examples of such probes include antibodies, a Fab of an antibody, a scFv, a soluble engineered TCR or an aptamer. The CAR may be able to directly recognise the probe or the probe may have a tag that is recognised by the CAR. In either regard the probe provides the specificity for the target cell (namely a cell having a dysfunctional P2X₇ receptor) whilst the genetically modified cell having the CAR provides the efficacy and directs an immune response against the target cell. Alternatively, the intermediate could be a cell endogenous marker which is associated with, or its expression is correlated to, the dysfunctional P2X₇ receptor. The dysregulation of the marker may be a cause of or a result of the dysfunction of the P2X₇ receptor.

In some embodiments of the sixth aspect of the invention, the method of treating or preventing cancer in a subject further includes the step of providing the subject with an intermediate.

In some embodiments of the sixth aspect of the invention, the intermediate is a probe that binds to a dysfunctional P2X₇ receptor and the chimeric antigen receptor recognises the probe. Preferably the probe is an antibody or an aptamer.

The term "aptamer" as used throughout the specification refers to any oligonucleic acid, polynucleic acid, peptide or polypeptide which specifically binds to, or preferentially forms a complex with, a target (specifically a dysfunctional P2X₇ receptor).

In some embodiments of the sixth aspect of the invention, the probe includes a tag and the chimeric antigen receptor recognises the tag. Examples of a CAR that recognise cells by way of an intermediate are known in the art, for example European patent application EP 2651442.

Whilst the provision of a genetically modified cell expressing a CAR directed against a target cell having a dysfunctional P2X₇ receptor may be sufficient to provide effective immunotherapy against precancerous or cancerous cells, the provision of adjuvants together with the genetically modified cells may further enhance the induction of the immune response and may augment the immunotherapy. Cytokines, preferably proinflammatory cytokines, are particularly suitable adjuvants for provision to a subject together with genetically modified cells having CARs.

Therefore, in some embodiments of the sixth aspect of the invention, the genetically modified cell is administered to the subject together with a cytokine. It is to be understood that as used throughout the specification the term "together with" includes the genetically modified cell being administered simultaneously with a cytokine or administered in combination with a cytokine. Consequently, when administered in combination with a cytokine this may be considered to include a combination therapy whereby a subject's immunotherapy includes both treatment with a cytokine and treatment with a genetically modified cell having a CAR directed against a target cell expressing a dysfunctional P2X₇ receptor. In some forms, the cytokine is administered on a different day (>24hrs) to the administration of the genetically modified cells. In other forms the cytokine is administered on the same day (within 24hrs) as the genetically modified cells. In further forms the cytokine(s) and the genetically modified cell is administered within 18hrs, 12hrs, 6hrs, 4hrs, 2hrs, 1hr, 45mins, 30mins, 15mins, 10mins, 5mins, 2mins or 1min of each other.

Suitable cytokines for administration together with the genetically modified cell include IL-2, IL-4, IL-6, IL-7, IL-9, IL-12, IL-15, IL-17, IL-18, IL-21, IL-23, IFNα, IFNβ, IFNγ, GM-CSF, TGFβ and TNFα. Preferred cytokines include IL-2 and IFNα. Furthermore, the cytokines may be administered as recombinant forms, natural forms, or via delivery systems such as fusions with proteins, delivered as a nucleic acid sequence which is expressed in the genetically modified cell or conjugated with a polymer such a polyethylene glycol (PEG).

The cell to be genetically modified can be obtained from any suitable source. In some embodiments of the sixth aspect of the invention the cell to be genetically modified is an autologous cell, being a cell autologous to the cell expressing a dysfunctional P2X₇ receptor. Advantageously, an autologous cell would not be recognised as 'non-self' by the subject's immune system and would therefore be tolerated by the subject. However, in some forms of cancer suitable autologous cells may not be readily available. Therefore, in some embodiments of the invention the cell to be genetically modified is an allogeneic or heterologous cell.

P2X₇ dysfunction is a common molecular alteration in a variety of cancers. Consequently, the method of the sixth or seventh aspects of the invention can be used for the prevention and treatment of a variety of cancers.

In some embodiments of the sixth aspect of the invention the genetically modified cell is for use in the prevention or treatment of a cancer selected from one or more of; brain cancer, oesophageal cancer, mouth cancer, tongue cancer, thyroid cancer, lung cancer, stomach cancer, pancreatic cancer, kidney cancer, colon cancer, rectal cancer, prostate cancer, bladder cancer, cervical cancer, epithelial cell cancers, skin cancer, leukaemia, lymphoma, myeloma, breast cancer, ovarian cancer, endometrial cancer, testicular cancer. Preferably the cancer is selected from one or more of lung cancer, oesophageal cancer, stomach cancer, colon cancer, prostate cancer, bladder cancer, cervical cancer, vaginal cancers, epithelial cell cancers, skin cancer, blood-related cancers, breast cancer, endometrial cancer, uterine cancer testicular cancer..

In some embodiments of the sixth aspect of the invention, the cancer is metastatic cancer, such as stage III or stage IV cancer.

The delivery or administration of the genetically modified cell according to a fourth or fifth aspect of the invention may be delivery or administration of the cell alone, or delivery or administration of the cell formulated into a suitable pharmaceutical composition. Accordingly, in an seventh aspect, the present invention provides a pharmaceutical composition including a genetically modified cell according to a fourth or fifth aspect of the invention, and a pharmaceutically acceptable carrier.

Methods are known in the art for providing CAR-containing cells for immunotherapy (see for example Kershaw, MH. et al. Clin Cancer Res. 2006;12(20): 6106-15; Parker LL. et al. Hum Gene Ther 2000;11: 2337-87). Furthermore, protocols and methods are known in the art for the preparation, expansion and assessment of mammalian CAR-expressing cells (see for example Cheadle, EJ. et al. Antibody Engineering: Methods and Protocols, Second Edition, Methods in Molecular Biology, vol. 907: 645-66) and are summarised in the Examples below.

The pharmaceutical composition may also include one or more pharmaceutically acceptable additives, including pharmaceutically acceptable salts, amino acids, polypeptides, polymers, solvents, buffers, excipients and bulking agents, taking into consideration the particular physical and chemical characteristics of the cell to be administered. In some embodiments, the pharmaceutical composition includes a suspension of genetically modified cells according to the fourth or fifth aspects of the invention in a suitable medium, such as isotonic saline solution. In some embodiments, the pharmaceutical composition may include suitable adjuvants such as one or more cytokines as described above. In some embodiments, the pharmaceutical composition may also include an intermediate as described above.

Administration of the pharmaceutical composition may also be via parenteral means which include intravenous, intraventricular, intraperitoneal, intramuscular or intracranial injection, or local injections to the site of a tumour or cancerous mass.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

Finally, reference is made to standard textbooks of molecular biology that contain methods for carrying out basic techniques encompassed by the present invention. See, for example, Green MR and Sambrook J, Molecular Cloning: A Laboratory Manual (4th edition), Cold Spring Harbor Laboratory Press, 2012.

It will be apparent to the person skilled in the art that while the invention has been described in some detail for the purposes of clarity and understanding, various modifications and alterations to the embodiments and methods described herein may be made without departing from the scope of the inventive concept disclosed in this specification.

The invention is further illustrated in the following examples. The examples are for the purpose of describing particular embodiments only and are not intended to be limiting with respect to the above description

### EXAMPLE 1

### Protocol for PEP2-2-3 binding peptide Chimeric Antigen Receptor (CAR) Design and Expression

An exemplified protocol detailing the process of designing and expressing an anti-non-functional (nf) P2X₇ receptor CAR according to an embodiment of the present invention is detailed as follows.

### Design of PEP2-2-3 (anti-nf P2x₇) Chimeric Antigen Receptor

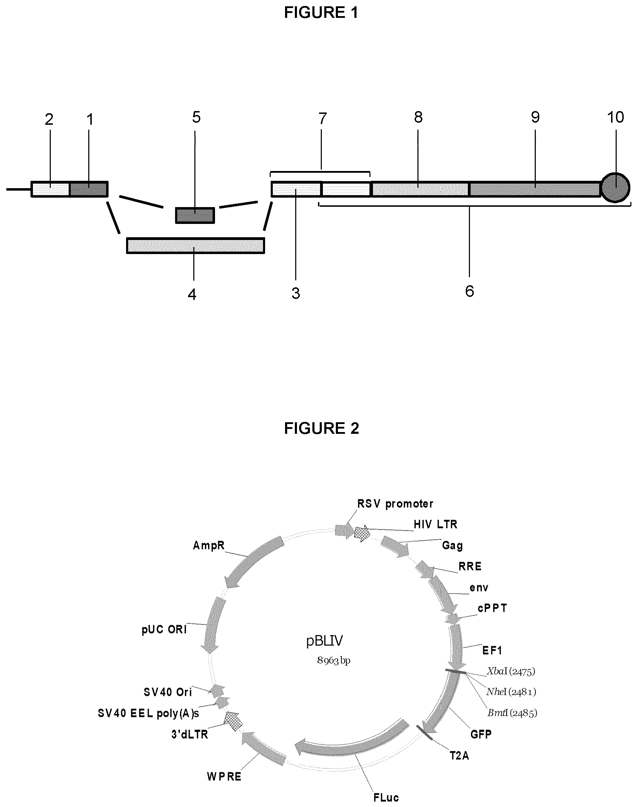

An anti-nfP2x₇ chimeric antigen receptor (CAR) was designed according to the schematic illustrated in Figure 1.

An antigen-recognition domain 1 of the CAR was generated which included the amino acid sequence of the PEP2-2-3 binding peptide (amino acid sequence set forth in SEQ ID NO: 10 and nucleotide sequence set forth in SEQ ID NO: 11). The PEP2-2-3 sequence was shown to have specific affinity for the dysfunctional P2X₇ receptor expressed on cancer cells, such as prostate LNCap cells, without significant affinity for monocytes or lymphocytes.

A CD8a signalling peptide **2** (having the amino acid sequence set forth in SEQ ID NO: 12 and the nucleotide sequence set forth in SEQ ID NO: 13) was linked to the N-terminus of the PEP2-2-3 antigen-recognition domain **1.** The CD8a signalling peptide **2** includes a Kozak consensus sequence at positions 1 to 13 of SEQ ID NO: 13. The CD8a signalling peptide **2,** including the Kozak sequence, acts to facilitate recognition of the transcribed RNA by the ribosome and provides a translation start site, thereby promoting translation of the transcribed RNA sequence of the CAR to a protein.

The antigen-recognition domain **1** of the CAR was linked to a transmembrane domain **3** via one of two hinge regions, termed long hinge **4** and short hinge **5.** The provision of a long hinge **4** permits for flexibility of the antigen-recognition domain which may be required for the antigen-recognition domain to interact with its cognate ligand (dysfunctional P2X₇). The amino acid and nucleotide sequences for the long hinge **4** are set forth in SEQ ID NO: 14 and SEQ ID NO: 15, respectively. The amino acid and nucleotide sequences for the short hinge **5** are set forth in SEQ ID NO: 16 and SEQ ID NO: 17, respectively.

The transmembrane domain **3,** and a portion of the intra-cellular domain **6,** of the CAR is provided by a portion of the CD28 co-stimulatory receptor **7** (amino acid sequence set forth in SEQ ID NO: 18 and nucleotide sequence set forth in SEQ ID NO: 19). The intracellular domain further includes a portion of the co-stimulatory receptor OX40 **8** (amino acid sequence set forth in SEQ ID NO: 20 and nucleotide sequence set forth in SEQ ID NO: 21) and a portion of the activation receptor CD3 zeta **9** (amino acid sequence set forth in SEQ ID NO: 22 and nucleotide sequence set forth in SEQ ID NO: 23).

A P2A sequence **10** (amino acid sequence set forth in SEQ ID NO: 24 and nucleotide sequence set forth in SEQ ID NO: 25) was added to the C-terminus of the CAR, permitting the post-translational excision of any peptide sequence attached to the C-terminus of the CAR. The amino acid sequence for the constructed anti-nfP2X₇ CAR-long hinge and the anti-nfP2X₇ CAR-short hinge are set forth in SEQ ID NOs: 26 and 27, respectively.

### Lentival Vector Design and Assembly

The designed CARs were incorporated into the BLIV lentiviral plasmid (System Biosciences, California, USA) illustrated in Figure 2, which includes the fluorescence and bioluminescence reporting proteins, green-fluorescence protein (GFP) and Firefly Luciferase (FLuc). The BLIV plasmid further includes a T2A coding sequence between the GFP and FLuc reporter protein coding sequences permitting for post-translational separation of the FLuc and GFP proteins.

Sequences having homology to the sequences upstream and downstream of the *Nhel* restriction site of the BLIV vector were added to the 5' and 3' ends of the designed CARs to result in the final nucleotide sequences set forth in SEQ ID NO 28 (CAR-long hinge) and SEQ ID NO: 29 (CAR-short hinge). The inclusion of the 5' and 3' sequences permitted incorporation of the anti-nf P2X₇ CAR into the BLIV vector using Gibson cloning.

The nucleotide sequence for the anti-nf P2X₇ CAR-long hinge and anti-nf P2X₇ CAR-short hinge were constructed using gene blocks technology (gBlock^{™} Gene Fragments - Integrated DNA Technologies, lowa, USA) and assembled using Gibson Assembly Cloning Kit (New England Biolabs inc. Ipswich MA, USA - cat# E5510S) in accordance with the manufacturer's instructions.

The BLIV plasmid was restricted at the *Nhel* cloning site and the anti-nf P2X₇ CAR coding sequence was incorporated using Gibson assembly.

### Cloning and evaluation of BLIV-CAR vector

New England Biolabs 5-alpha Competent *E. coli* cells (provided in Gibson Assembly Cloning Kit) were transformed with the generated BLIV-CAR vectors as per the manufacturer's instructions. Briefly:
- A tube of NEB 5-alpha Competent E. coli cells was thawed on ice for 10 minutes;
- 1-5 µl containing 1 pg - 100 ng of BLIV-CAR plasmid DNA was added to the cell mixture and mixed in by flicking the tube 4 to 5 times;
- The *E.coli* and plasmid mixture was placed on ice for 30 minutes without mixing;
- The cell and plasmid mixture were heat shocked at 42°C for 30 seconds before being placed on ice for 5 minutes without mixing;
- 950 µl of SOC was added to the mixture before being heated to 37°C for 60 minutes and shaken vigorously;
- Selection plates were prepared and heated to 37°C;
- Serial 10-fold dilutions of the cells were prepared in SOC solution; and
- 50 to 100 µl of each dilution was spread onto selection plates followed by incubation overnight at 37°C.

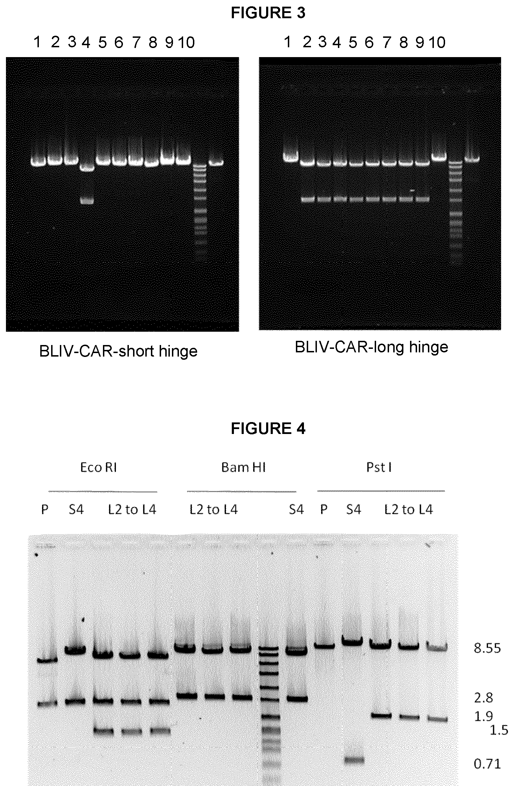

Following incubation of the transformed (*E.coli*) cells, 10 colonies of bacteria transformed with BLIV-CAR-short hinge plasmid and 10 colonies of bacteria transformed with the BLIV-CAR-Long hinge plasmid were isolated, plasmid DNA was purified, and restricted with a *Bam*HI restriction enzyme. The restricted DNA was analysed via gel electrophoresis for appropriate sized restriction fragments. As shown in Figure 3, colonies 2 to 9 of the bacterial clones transformed with the BLIV-CAR-long hinge plasmid contained the appropriately sized restriction fragments (7.8kb and 2.8kb), while only colony 4 of the bacterial clones transformed with the BLIV-CAR-short hinge plasmid provided the appropriate sized restriction fragments (7.4kb and 2.8kb).

Clones 2 to 4 of the bacteria containing the BLIV-CAR-long hinge plasmid (L2 to L4), and clone 4 of the bacteria containing the BLIV-CAR-short hinge plasmid (S4) were selected for further confirmation of plasmid identity using *Eco*RI, *Bam*HI and *Pst*I restriction enzymes. All of the colonies showed restriction fragments of the expected length, as set out in Table 4 and Figure 4.

**TABLE 4**

| **Expected Restriction Fragment Length of BLIV-CAR Plasmids** | |
|---|---|
| **Restriction Enzyme and Plasmid** | **Expected Length** |
| Unmodified BLIV plasmid | 8.9kb |
| Bam HI restricted BLIV-CAR-long hinge | 7.8kb and 2.8kb |
| Bam HI restricted BLIV-CAR-short hinge | 7.4kb and 2.8kb |
| Eco RI restricted BLIV-CAR-long hinge | 6.8kb, 2.6kb and 1.5kb |
| Eco RI restricted BLIV-CAR-short hinge | 7.7kb and 2.6kb |
| Pst I restricted BLIV-CAR-long hinge | 8.6kb, 2.0kb and 0.22kb |
| Pst I restricted BLIV-CAR-short hinge | 9.3kb, 0.8kb and 0.22kb |

### Construction and Verification of Lentiviral Vectors

293T cells were used to package lentivirus from a 3 plasmid protocol according to the following method.

**Day 1:** 293T cells were seeded in 35ml DMEM media with 10% serum in a T-225 flask such that the cells were 90-95% confluent the following day.

**Day 2:** 30ug of one of the generated BLIV-CAR plasmids (or an unmodified BLIV plasmid), 30ug of gag-pol plasmid delta 8.2, and 15ug of VSV-G plasmid (pMD2.G), were added to OptiMEM media to a final volume of 750ul, and mixed. 300ul of PEI solution were added and incubated at room temperature for at least 20 minutes. The mixture was then added to the confluent 293T cells before incubation at 37°C.

**Day 3:** Supernatant was decanted from the 293T cells 24 hours after addition of the plasmid mixture and stored at 4°C. The decanted mixture was replaced with 35ml of fresh media before further incubation at 37°C.

**Day 4:** 48 hours after addition of the plasmid mixture, the media was removed and combined with the supernatant from the 24 hour harvest. The combined supernatants were spun for 15 minutes at 1500g to remove any remaining cellular debris. The supernatant was filtered through a 0.45um filter, and then spun at 17,000rpm in a WX ultracentrifuge for one hour. After centrifugation, the supernatant was decanted by hand, with 50-200ul remaining in the tube. The centrifuge tube was placed in a 50ml screw-top tube in order to prevent contamination and evaporation and the virus was allowed to resuspend at 4°C overnight.

**Day 5:** The virus was resuspended off the bottom of the centrifuge tube and transferred into a new 1.5ml tube. The resuspended virus was spun for 5 minutes in a microcentrifuge tube at 5000 rpm to remove any remaining debris.

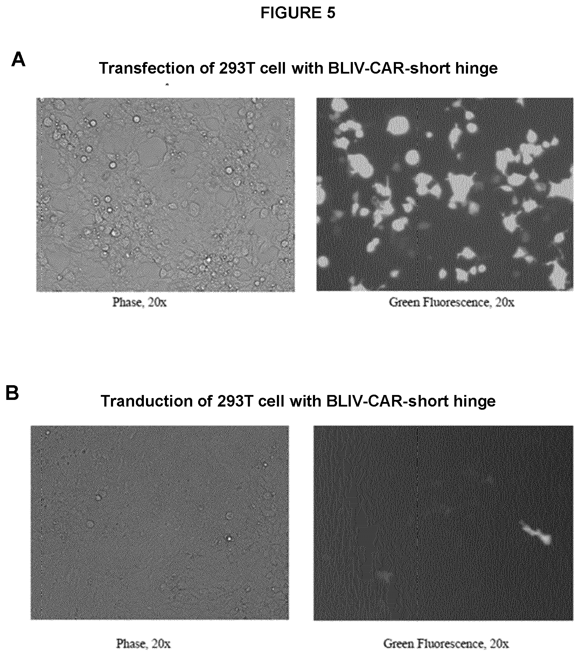

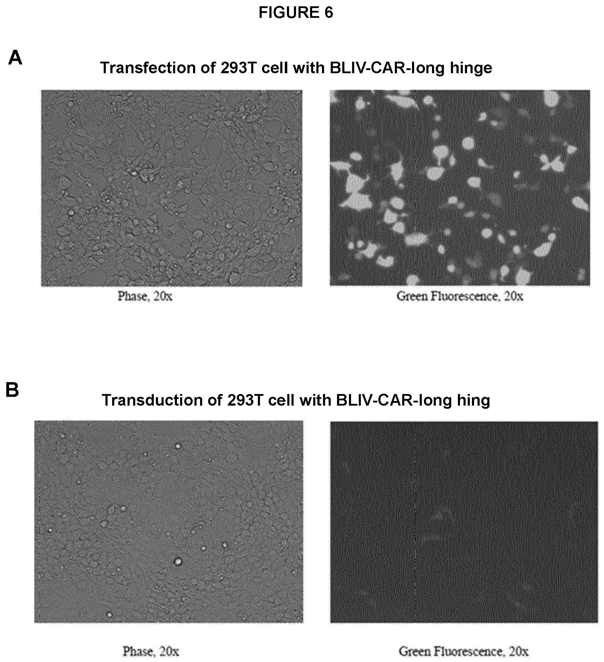

Transfection of 293T cells with the BLIV-CAR-short hinge and BLIV-CAR- long hinge vector was assessed after 24 hours of incubation by the presence of GFP fluorescence (see Figure 5A and Figure 6A). Supernatant collected at day 5 (as set out above) containing short- and long-hinge BLIV-CAR lentivirus vectors were incubated with fresh 293T cells and visualized for GFP fluorescence to test transduction capacity (see Figure 5B and Figure 6B).

### Screening for CAR T cell function

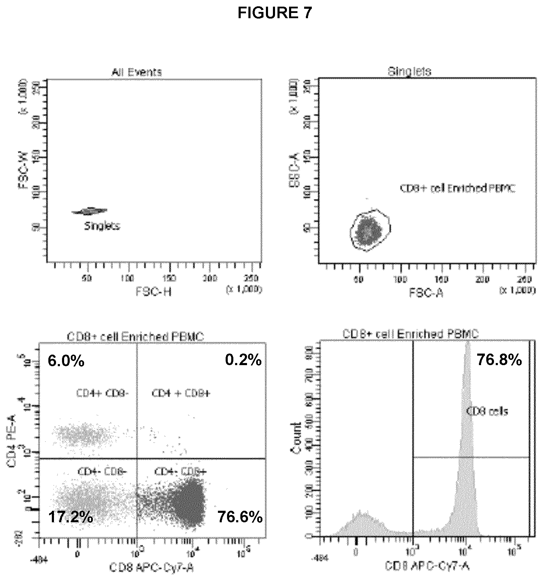

10⁸ CD8 T cells were isolated from 50ml of human blood using the RosetteSep^{™} human CD8+ T cell isolation kit (Stemcell technologies, Vancouver, Canada) in accordance with the manufacturer's instructions. Analysis of the purity, as illustrated in Figure 7, demonstrated that 76.6% of purified cells were CD8+

CD8+ T cells were incubated at 10⁵ cells per well with a 1:1 ratio of dynal T cell expander (CD3/CD28) beads. The CD8 cells were then incubated overnight together with lentiviral preparations, at a multiplicity of infection (MOI) of 5 or greater, containing either unmodified BLIV plasmids, BLIV-CAR-short hinge plasmids or BLIV-CAR-long hinge plasmids. Following incubation, the CD8+ T cells were washed before being co-cultured with the target cells.

Target cells expressing the non-functional P2X₇ receptor were provided by the mammary cancer cell line BT549 (ATCC HTB-122). These cells were dye-labelled using the fluorescent membrane intercalculating dye eFluor^{™} 670 (affymetrix eBioscience) as per the manufacturer's instructions. Briefly:
- BT549 cells were prepared as a single-cell suspension and washed in PBS twice to remove any residual serum;
- Cells were resuspended in room temperature PBS;
- A 10 µM solution of Cell Proliferation Dye eFluor^{®} 670 was prepared in room temperature PBS;
- An equal volume of the 10 µM dye solution was added to the prepared BT549 cells to give a final concentration of 5 µM dye solution;
- The BT549 cells in the dye solution were incubated for 10 minutes at 37°C in the dark, before the labelling was stopped by adding 4 times the volume of cold culture medium containg 10% serum followed by incubation on ice for 5 minutes in the dark;
- Finally cells were washed 3 times in culture medium before being resuspended in culture medium at the desired concentration.

Following dye labelling, target cells were co-culturing with the prepared CD8+ T cells at ratios of 10:1, 5:1, 1:1 and 0:1 (T cells:targets).

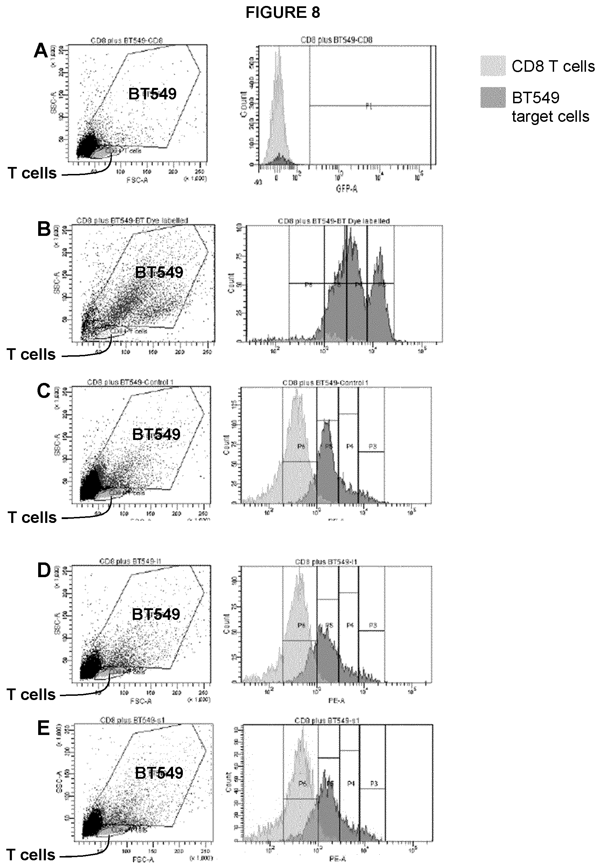

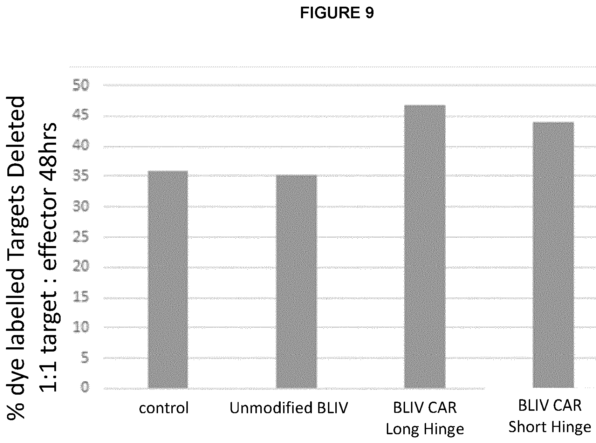

After 24hrs of co-culture, cells were collected and analysed using Fluorescence-Activated Cell Sorting (FACS). The number of target cells containing the membrane intercalculating dye was quantified to assess if the co-cultured T cells led to target cell death or arrest of cell proliferation. The gating and analysis strategy used to quantify the efficacy of the CD8+ T cells at killing target cells is illustrated in Figure 8, and is quantified in Figure 9. Figure 8A illustrates the gating and histogram analysis of labelled CD8+ T cells. Figure 8B illustrates the gating and histogram analysis of labelled BT549 target cells. Figure 8C illustrates the gating and histogram analysis after 24hrs of co-culture of control CD8+ T cells and BT549 targets. Figure 8D illustrates the gating and histogram analysis after 24hrs of co-culture of CD8+ T cells transduced with BLIV-CAR-long hinge and BT549 target cells. Figure 8E illustrates the gating and histogram analysis of 24hr co-culture of CD8+ T cells transduced with BLIC-CAR-short hinge and BT549 target cells.

As can be seen in Figure 9, there was an increase in the number of BT549 target cells deleted (killed) when the target cells were co-cultured with CD8 T cells transduced with lentivirus containing either the BLIV-CAR-long hinge or BLIV-CAR-short hinge, compared to the co-culture of the target cells with non-transduced or control transduced (unmodified BLIV vector) CD8 T cells.

In view of the results presented in Figure 9, it is apparent that CD8+ T cells transduced with anti-nfP2X₇ CAR receptors (having either the short or the long hinge) demonstrate elevated levels of cytotoxic activity towards non-functional P2X₇ expressing target cells, demonstrating the ability of the CAR-T cells to kill cancer cell targets.

### EXAMPLE 2

### Design of alternative anti-nfP2X7 Chimeric Antigen Receptor

A further exemplified protocol detailing the process of designing, and expressing on a T cell, an anti-non-functional (nf) P2X₇ receptor CAR, according to an embodiment of the present invention, is detailed below.

Anti-nfP2X₇ CARs were designed utilising three anti-non-functioning P2X₇ binding peptides. Specifically, CARs were designed to include antigen recognition domains with sequence homology to peptides PEP2-2-1-1, PEP2-472-2 or PEP2-2-12 (having the amino acid sequences set forth in SEQ ID NOs: 32, 33 and 34 respectively). These binding peptides have been shown to bind to the non-functional P2X7 receptor (Barden, J.A., Sluyter, R., Gu, B.J. & Wiley, J.S. 2003. Specific detection of non-functional human P2X(7) receptors in HEK293 cells and B-lymphocytes. FEBS Lett 538, 159-162).

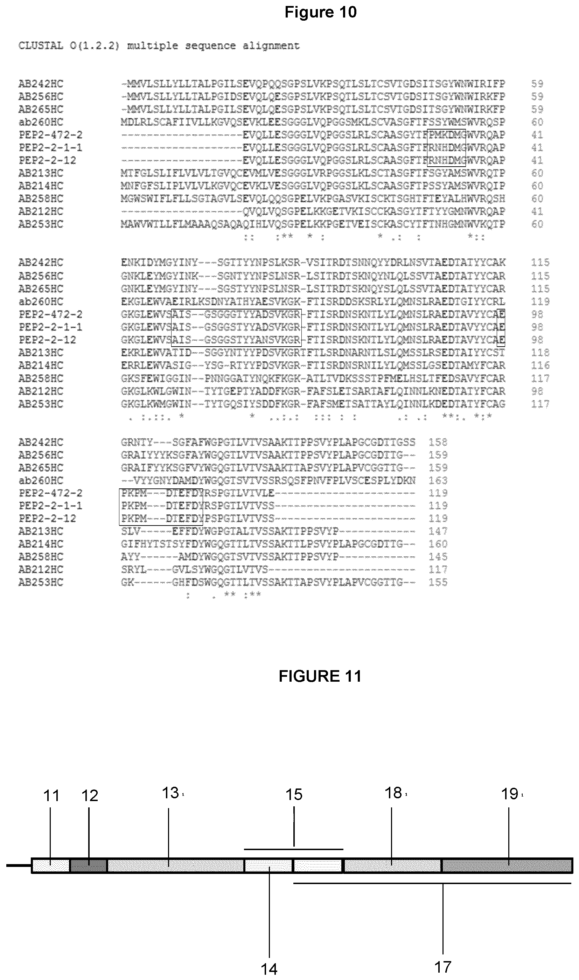

The alignment of the above binding peptides to the heavy chain variable regions of antibodies that recognise non-functional P2X₇ receptors is shown in Figure 10. The alignment of the Complementarity Determining Region (CDR 1 to 3) sequences are indicated by the boxes.

A specific example of the construction of a CAR having the PEP2-2-1-1 sequence is detailed below. The same CAR structure and sequences were used for CARs having PEP2-472-2 or PEP2-2-12 sequences as the binding peptides, with the alternative binding peptides substituted for PEP2-2-1-1.

DNA sequences coding for the PEP2-2-1-1 binding peptide were synthesised in-frame with other DNA sequences to generate a CAR having the configuration described below.

With reference to Figure 11, an antigen recognition domain was prepared by linking a leader sequence of Homo sapiens CD8a molecule (CD8A) transcript variant 1 (having the amino acid sequence set forth in SEQ ID NO: 30 and the nucleotide sequence set forth in SEQ ID NO: 31) **11** to the N terminus of the PEP2-2-1-1 binding peptide 12 (having the amino acid sequence set forth in SEQ ID NO: 32 and the nucleotide sequence set forth in SEQ ID NO: 35).

The antigen recognition domain was then linked to a transmembrane domain via a modified IgG4 Hinge-CH2-CH4 13 having the sequence of the long hinge as set out in Example 1 above (i.e. the amino acid sequence set forth in SEQ ID NO: 14 and the nucleotide sequence set forth in SEQ ID NO: 15).

The extracellular domain comprising the CD8 leader sequence **11** and the PEP2-2-1 binding peptide **12** was linked to a transmembrane domain **14** provided by a portion of human CD28 **15** (having the amino acid sequence set forth in SEQ ID NO: 18 and the nucleotide sequence set forth in SEQ ID NO: 19) which also included a portion of the CD28 cytoplasmic domains **16.**

The intracellular portion of the CAR **17** was provided by a portion of the human CD28 molecule mentioned above **14** and the cytoplasmic domain of Homo sapiens tumour necrosis factor receptor superfamily member 4 (TNFRSF4/OX40 - having the amino acid sequence set forth in SEQ ID NO: 20 and the nucleotide sequence set forth in SEQ ID NO: 21) **18** linked to the cytoplasmic domain of Homo sapiens CD247 molecule **19** (T-cell surface glycoprotein CD3 zeta chain, having the amino acid sequence set forth in SEQ ID NO: 22 and the nucleotide sequence set forth in SEQ ID NO: 23).

### Lentival Vector Design and Assembly

The nucleotide sequences for the designed PEP2-2-1-1, PEP2-472-2 and PEP2-2-12 CARs were constructed using gene blocks technology (gBlock^{™} Gene Fragments - Integrated DNA Technologies, lowa, USA) and assembled using Gibson Assembly Cloning Kit (New England Biolabs inc. Ipswich MA, USA - cat# E5510S) in accordance with the manufacturer's instructions. The sequences of the nucleotide constructs for PEP2-2-1-1, PEP2-472-2 or PEP2-2-12 CARs for integration in to cloning vectors (including restriction sites) are set out in SEQ ID NOs: 35, 36 and 37, respectively.

The CAR nucleotide constructs were incorporated into the pCDH-CMV-MCS-T2A (pCDH) vector (System Biosciences, California, USA Cat# CD524A-1) illustrated in Figure 11, which includes the fluorescence reporting protein, green-fluorescence protein (GFP). The pCDH vector further includes a T2A coding sequence between the cloning site and the GFP permitting for post-translational separation of the cloned CAR and the GFP proteins.

For integration of PEP2-2-12 and PEP2-472-2 CAR nucleotide constructs into the pCDG vector, the pCDH vector was restricted with *EcoR1* and *NotI* and gel purified (QIAquick gel extraction kit, QIAGEN). PEP2-2-12 and PEP2-472-2 CAR nucleotide gBlock constructs were also digested with *EcoRI* and *NotI* digestion enzymes. The restricted gBlock fragment was then purified with a QIAquick PCR purification kit in accordance with the manufacturer's instructions. The restricted vector was ligated with the restricted CAR constructs at a 3:1 molar ratio of insert to vector. Ligation mixes were transformed into chemical competent SURE2 cells (Agilent).

The PEP2-2-1-1 CAR construct contained an internal *EcoR1* restriction site and therefore it was integrated into the pCDH vector in a manner different to the PEP2-2-12 and PEP2-472-2 CAR nucleotide constructs. The pCDH vector was restricted with *EcoR1,* and the resulting 5'-overhang filled by T4 DNA polymerase in the presence of 100uM dNTPs (12°C for 15 minutes). The reaction was terminated (75°C for 20 minutes in the presence of 10mM EDTA) and the restricted vector was column purified (QIAquick PCR purification kit, QIAGEN). The purified vector was then further restricted with *Not*I and gel purified (QIAquick gel extraction kit, QIAGEN). The PEP2-2-1-1 CAR construct fragment was first restricted with *Smal* followed by *Not*I digestion (both at 25°C). The restricted gBlock fragment was purified with a QIAquick PCR purification kit in accordance with manufacturer's instructions. The restricted vector was ligated with the CAR construct at a 3:1 molar ratio of insert to vector.

### Cloning and evaluation of pCDH-CAR vector

Ligation mixes for each of the three CAR constructs described above were transformed into chemical competent SURE2 cells (Agilent) in accordance with the manufacturer's instructions. Briefly:
- SURE2 cells were thawed on ice. Once thawed, the cells were gently mixed and 100 µl aliquots of cells were placed in pre-chilled 14ml round bottom tubes;
- 2µl of the β-Mercaptoethanol was added to each aliquot of cells;
- The tubes were mixed and incubated on ice for 10 minutes, swirling gently every 2 minutes;
- 0.1-50ng of each of the pCDH-CAR vectors was added to an aliquot of cells;
- The aliquots were gently mixed, then incubate on ice for 30 minutes;
- The tubes were heat-pulsed at 42°C in a water bath for 30 seconds and then incubate on ice for 2 minutes;
- 0.9ml of preheated (42°C) NZY+ broth was added to each tube followed by incubation at 37°C for 1 hour with agitation at 225-250 rpm;
- Up to 200µl of the transformation mixture was plated on LB agar plates containing antibiotic, followed by incubation at 37°C overnight;
- Colonies were picked and cultured further overnight;
- Plasmid DNA was isolated from cultured clones with a Quicklyse miniprep kit (QIAGEN) and digested with *EcoRI*/*Not I* digestion to identify clones with the correctly sized CAR-pCDH vectors

Following incubation of the transformed (SURE2) cells, 5 to 6 colonies of cells transformed with pCDH-CAR for each of the PEP2-2-1-1, PEP2-472-2 or PEP2-2-12 binding peptides were isolated and further incubated overnight. Plasmid DNA was isolated with a Quicklyse miniprep kit (QIAGEN) from each of the cultured colonies, and restricted with the *EcoRI*/*Not I* restriction enzymes. The restricted DNA was analysed via gel electrophoresis for appropriate sized restriction fragments.

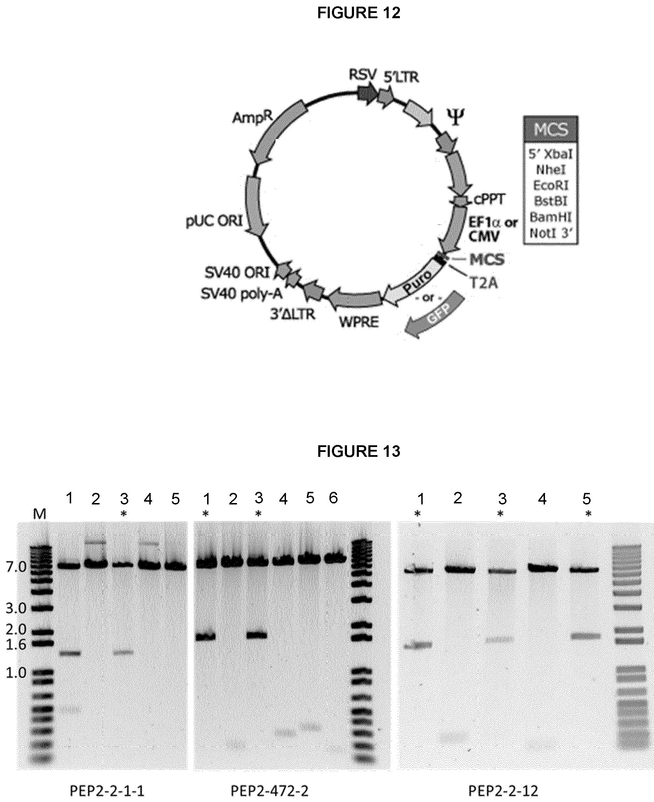

As shown in Figure 13, colony 3 of the PEP2-2-1-1 pCDH-CAR construct, colonies 1 and 3 of the PEP2-472-2 pCDH-CAR construct and colonies 1, 3 and 5 of the PEP2-2-12 pCDH-CAR construct contained the appropriately sized restriction fragments.

Each selected clone was sequenced to confirm the integration of the CAR using the appropriate primers selected from Table 5.

**TABLE 5**

| **Primers used for confirmation of correct CAR construct in selected colonies** | | |
|---|---|---|
| **Primer name** | **Sequence** | **SEQ ID NO** |
| pCHD-CMV-For | GGTGGGAGGTCTATATAAGC | SEQ ID NO: 38 |
| pCHD-coGFP-Rev | TGATGCGGCACTCGATCTC | SEQ ID NO: 39 |
| 2-2-1-1-Rev | CTTCACGGAGTCTGCGTAG | SEQ ID NO: 40 |
| 2-2-1-1-For | TCTTGTCACTGTATCCAGTG | SEQ ID NO: 41 |
| 2-472-2-Rev | CGTATCTTCAGCTCTCAAGC | SEQ ID NO: 42 |
| 2-472-2-For | TGGTCCTTCAGTTTTCCTGT | SEQ ID NO: 43 |
| 2-12-2-Rev | CAGCTGTATCTTCTGCTC | SEQ ID NO: 44 |
| Com-For-1 | AGTGGGAGAGTAACGGACAG | SEQ ID NO: 45 |
| Com-For-2 | AGGGCCAGAATCAATTGTAC | SEQ ID NO: 46 |

Sequencing data for each selected colony was aligned to the *in silico* derived recombinant clone for each of the PEP2-2-1-1, PEP2-472-2 or PEP2-2-12 CAR constructs and appropriate constructs were verified for at least one of each of the selected colonies. Large scale Endotoxin free plasmid isolation of the verified clones was performed with a NucleoBond^{®} Xtra Midi EF kit, Macherey-Nagel in accordance with the Manufacturer's instructions.

### Construction and Verification of Viral Vectors

Lentivirus packaging was performed in transiently transfected Hek293T cells using Lipofectamine 2000 reagent (Invitrogen) as per a standard laboratory protocol (Brown, C.Y. et al. 2010. Robust, reversible gene knockdown using a single lentiviral short hairpin RNA vector. Hum Gene Ther 21, 1005-1017). Briefly:
- 12.5ug of Lentiviral vector DNA was mixed with 3.75ug of pMD2.g (VSV-G envelope expression vector), 6.25ug of pRSV-Rev and 7.5ug of pCMVdelta8.2 per transfection in a T75cm flask using 75ul of Lipofectin as per the manufacturer's protocol, and incubated overnight;
- The following morning media was changed and virus containing supernatant was harvested 48 hours later;
- Harvested supernatants were centrifuged at 300xg for 5 minutes before being filtered through a 0.45um filter;
- Virus particles from filtered supernatants were concentrated by ultracentrifugation (68,000xg for 90 minutes and 4°C, Beckman SW32 rotor). The supernatant was removed and the virus pellet resuspended gently in DMEM on ice;
- 100ul virus aliquots were stored at -70°C until needed.

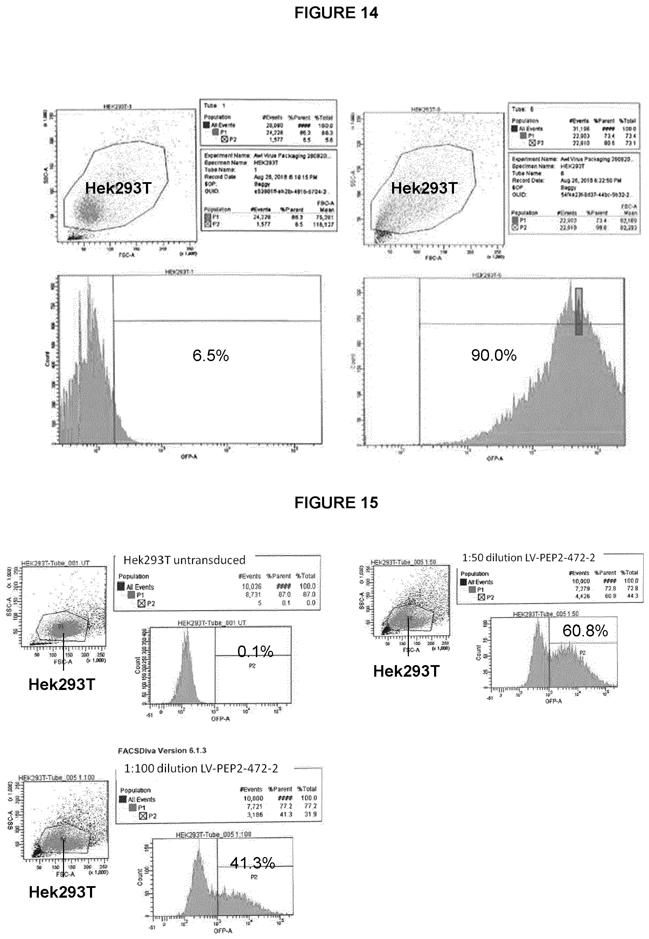

To assess the rate of virus transfection, transfected Hek293T cells were harvested and the percent of GFP positive cells (pCDH vector containing cells) was determined by flow cytometry. Representative results for Hek293T transfected with the LV-PEP2-472-2 packaging mix is shown in Figure 14.

Viral titres were calculated by transduction of a known number of Hek293T cells with serial dilutions (1:50 and 1:100) of concentrated LV stock. Transductions were performed overnight in the presence of 8ug/ml polybrene (Hexadimethrine bromide). The following day media containing virus and polybrene was replaced with fresh media, cells were harvested 24 hours later and the percentage of GFP positive cells determined by flow cytometry. Viral titres were calculated using the formula: Transduction units/ml (TU) = (FxC/V)xD where F= frequency of GFP+ cells (%GFP+/100), C= cell number at the time of virus addition, V= volume of transduction in mL and D= dilution factor. Representative flow data for LV-PEP2-472-2 transduction is shown in Figure 15. The TU for each of the PEP2-2-1-1, PEP2-12-2 and PEP2-472-2 CAR viral vectors is provided in the Table 6 below.

**TABLE 6**

| **Transduction Units for Viral Vectors** | | | |
|---|---|---|---|
| **Lentiviral construct** | **Dilution Factor** | **Average %GFP+ cells** | **TU/ml** |
| PEP2-2-1-1 | 50 | 31.15 | 4.14e6 |
| | 100 | 21.25 | 5.62e6 |
| PEP2-12-2 | 50 | 54.6 | 7.26e6 |
| | 100 | 33.15 | 8.82e6 |
| PEP2-472-2 | 50 | 62 | 8.25e6 |
| | 100 | 39.55 | 10.52e6 |

### Screening for nf-P2X₇ CAR T Cell Function

### Production of CD8 T Cells Expressing Anti-nf-P2X₇ CARs

Human CD8 cells were purified and transduced in accordance with the following method:

Human CD8 T cells were purified from mononuclear cells (MNCs) isolated from Buffy Coats from anonymous donors (Australia Red Cross blood service). MNCs were isolated using Ficoll-Paque^{™} density gradient media. CD8 T cells were purified from MNCs Dynabeads^{®} Untouched^{™} Human CD8 T Cells Kit (Invitrogen) according manufacturer's instructions. The purity of isolated cells, as assessed by flow cytometry, was ≥85%.

2x10⁶ purified cells were pre-incubated with CD3/CD28 beads (3:1 bead to cell ratio) and IL2 (500U/ml) for 30 minutes prior to the addition of 1 to 2 multiplicity of infection (MOI) units of virus containing LV-PEP2-2-1-1, LV-PEP2-472-2 or an empty LV vector (GFP control virus) together with 8ug/ml polybrene. Cells were incubated with virus for 16 hours before the virus containing media was removed. The remaining cells and beads were incubated in fresh media including IL2 for 40 hours before GFP fluorescence levels were analysis.

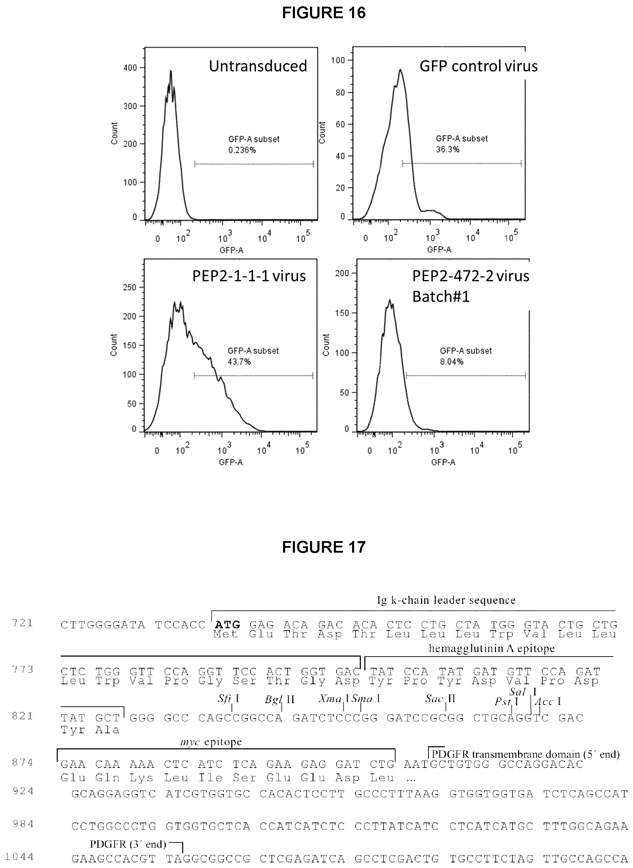

As illustrated in Figure 16, between 8% and 43% of CD8 cells GFP+ indicating successful transduction.

### Production of Target Cells expressing nf-P2X₇ or Wild-Type (WT) P2X₇ Receptors

To assess the efficacy of CD8 cells expressing the anti-nf-P2X₇-CARs, Hek293T cells over-expressing either a non-functional P2X₇ receptor (having a K193A mutation) or a wild type extracellular domain of the P2X₇ receptor on their cell surface, were prepared.

EXD2_K193A (nf-P2X₇) and EXD2_WT (functional P2X₇) gBlock gene fragments (SEQ ID NOs: 47 and 48, respectively) were ordered from Integrated DNA technologies (IDT). The EXD2 domains were designed to be expressed in frame with DNA sequences encoding for a fusion protein consisting of the IgK-leader-HA-MYC-PDGFR-transmembrane domian from pDisplay (Invitrogen - Figure 17). These fusion proteins were designed for surface expression. The EXD2_K193A and EXD2_WT gene fragments were cloned between the HA and MYC-epitope Tags to forma fusion gene blocks. Gateway attB1 and attB2 sequences were included at the 5'- and 3'- ends of the fusion gene blocks for cloning into the LV-416-IRES-puro vector (Clontech).

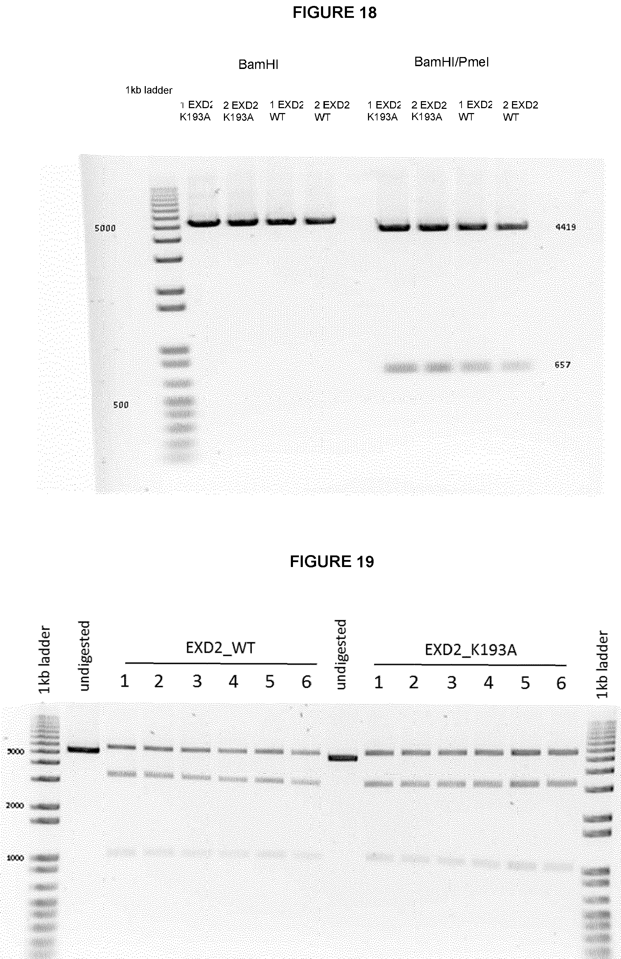

Cloning was performed using Gateway^{®} (ThermoFisher) and all steps were carried out following the manufacturer's protocol. Briefly:
- First BP recombination reactions were performed between attB-flanked DNA fragments (EXD2_K193A, SEQ ID NO: 47 and EXD2_WT, SEQ ID NO: 48) and an attP containing pDONR-107 vector to generate an entry clone. The BP recombination reactions were used to transform chemically competent E.cloni^{®}10G cells (Lucigen^{®}) according to manufacturer's protocol;
- Transformed cells were plated onto LB agar plates containing 50ug/ml Kanamycin (Sigma) and incubated at 37 °C overnight;
- Two clones from each plate were picked to prepare mini cultures (2 mL) in LB broth with kanamycin (SIGMA) (50ug/ml). Followed by incubation at 37°C overnight with agitation.
- Plasmid DNA was extracted the following day from the mini cultures using the QIAGEN QuickLyse miniprep kit;
- A diagnostic *Bam H1*-HF (NEB) and *Pmel* (NEB) digests were performed to identify recombinant clones. Both the EXD2_K193A and EXD2_WT clones were confirmed to be digested correctly via *Bam H1* and *Bam H1*/*Pmel* digestion followed by gel electrophoresis (Figure 18).

One clone from each construct (EXD2_K193A and EXD2_WT) was chosen for the LR recombination reaction (as set forth below) to insert the EXD2_K193A and EXD2_WT constructs into a pLV-416 destination vector.

Following selection of clones, LR recombination reactions were then performed to transfer each EXD2 insert from the pDONR-107 entry clone to the pLV-416 destination vector to create an expression vector. The final LR recombination reaction was used to transform chemically competent E.cloni^{®}10G cells (Lucigen^{®}) according to manufacturer's protocol. Briefly:
- Transformed cells were plated onto LB agar plates containing 100ug/ml Ampicillin (SIGMA) and incubated at 37 °C overnight;
- Six clones from each plate were picked to prepare mini cultures (2 mL) in LB broth with Ampicillin (50ug/ml), which were incubated at 37 °C overnight with agitation;
- The following day plasmid DNA was isolated and *Bam H1* digestion was performed to identify recombinant clones. Recombinant clones were identified by the presence of three bands of the appropriate size (3431, 1056 and 5844 bp - see Figure 19). As can be seen in Figure 19 all six selected clones from each plate provided the appropriate sized restriction fragments;
- Two clones transduced with the pLV-416 constructs containing EXD2_K193A or EXD2_WT were sequenced with the primers set forth in Table 7 to confirm the constructs were correct.

**TABLE 7**

| **Primers Used for Confirmation of Correct EXD2_K193A and EXD2_WT Constructs in Selected Colonies** | | |
|---|---|---|
| **Primer name** | **Sequence** | **SEQ ID NO** |
| EXD-F1 primer | ACAAGCTGTACCAGCGGAAA | SEQ ID NO: 49 |
| EXD2-R1 primer | CACCACCACCTTAAAGGGCA | SEQ ID NO: 50 |
| EXD2-F1 primer | ACAAGCTGTACCAGCGGAAA | SEQ ID NO: 51 |

To produce viral particles for transduction of HEK293 cells, and the generation of a stable HEK293 cell line, expressing a functional or non-functional P2X₇ receptor, the following protocol was used:
- HEK293 cells were plated (7x10⁶ cells per flask) a day prior to transfection.
- HEK293T cells were transfected with Lentiviral packaging vectors and either pLV-416-EXD2 and pLV-416-EXD2_WT. To monitor transfection efficiency, a GFP expression plasmid (1ug) was also included.
- Following overnight incubation, the medium containing the transfection reagents was removed and replaced with 10 ml of fresh medium (DMEM with 10% FCS). 10 ml of media was harvested 24 hours later and stored in 2 ml aliquots at -80°C until required. Another 10 ml of fresh medium (DMEM with 10% FCS) was added to the flasks which was harvested a further 24 hours later.
- Viral particles were isolated from the harvested media by centrifuging the media at 1200 rpm followed by filtration through a 0.45um filter. The filtered media, with virus particles, were used for transfection of HEK293 cells.

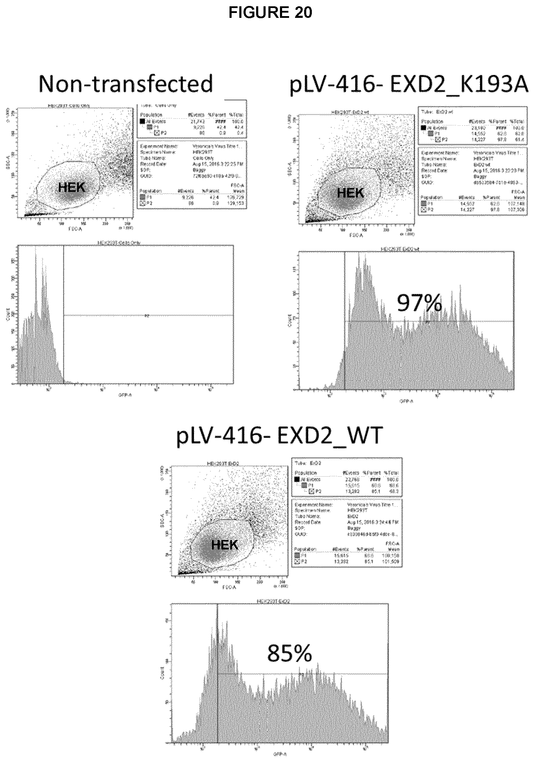

To assess transfection efficiency, cells were harvested following the removal of the second 10ml of medium and the percentage of GFP positive cells was determined by flow cytometry. Figure 20 illustrates that HEK293 cells were transfected with pLV-416-EXD2_K193A and pLV-416-EXD2_WT at an efficiency of 97% and 85%.

To generated stable HEK293 cells over-expressing the extracellular domain of functional and non-functional P2X₇ on their cell surface. The following protocol was used:
- HEK293 cells were plated (7x10⁵ per flask) in T25 flasks a day prior to transduction.
- The following day the medium was removed from each flask, and fresh media containing virus particles produced in accordance with the above protocol were added according to the ratios set out in Table 8;
- Polybrene was added to each flask to a final concentration of 8 ug/mL.

**Table 8**

| **Transduction Protocol** | | | | | | |
|---|---|---|---|---|---|---|
| Virus particle | Media | Media with virus | Media | Media with virus | Media | Media with virus |
| pLV-416-EXD2 | 2.5 mL | 2.5 mL | 4 mL | 1 mL | 4.5 mL | 0.5 mL |
| pLV-416-EXD2_WT | 2.5 mL | 2.5 mL | 4 mL | 1 mL | 4.5 mL | 0.5 mL |
| Control LV-411-GFP | 4 mL | 1 mL (0.5 MOI) | | | | |
| Polybrene 20 mg/mL stock | 2 uL | | 2 uL | | 2 uL | |
| Un-transduced | 5 mL | | | | | |

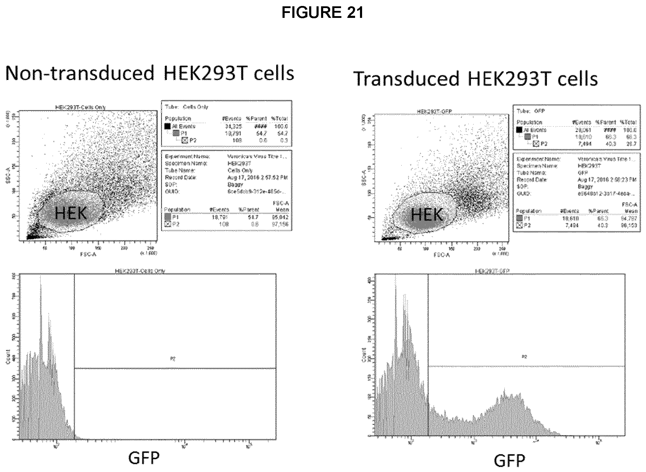

- 24 h after the transduction, the medium was removed from each flask and fresh medium (DMEM with 10% FCS) supplemented with 1600 ug/mL G418 was added to all flasks except the flask containing control Lentivirus expressing GFP (LV-411-GFP).
- HEK293T cells transduced with control pLV-411-GFP virus were monitored for GFP expression 72 hours post-transduction (see Figure 21);
- All un-transduced cells died 4 days after culturing with G418 supplemented media. The transduced cell lines continued to grow normally with G418 in media.

The extracellular domain of the transfected P2X₇ receptors contains HA- and MYC-epitope tags. Therefore, these cells can be stained with monoclonal antibodies against HA- and MYC- to confirm surface expression of the extracellular domain by flow cytometry.

### Screening for CAR T cell function

To assess the functionality of the nf-P2X7-CARs, CD8 cells transduced with each of the PEP2-2-1-1 or PEP2-472-2 CAR constructs (as prepared above) were co-incubated for 4 hours in a 96-well round-bottom culture plate at 1:1 ratio with 1X10⁴ target cells expressing an nf-P2X₇ receptor (as prepared above) and MDA-MB-231 breast cancer cells, which express a non-functional P2X₇ receptor (231 P2X₇ cells).

The percentage of cytotoxicity was determined via a in a CytoTox 96^{®} NonRadioactive Cytotoxicity Assay (Promega, Madison, Wisconsin, USA) in accordance with the manufacturer's instructions. Briefly:
- 45 mins prior to 4 hours, 10µl of Lysis Solution (10X) was added to each well for every 100µl of target cells;
- After a further 45 minutes the plates were centrifuge at 250xg for 4 minutes;
- 50µl aliquots were taken from each well and transferred to a 96-well flat-bottom plate;
- 50µl of CytoTox 96^{®} Reagent was added to each well of the plate containing the transferred aliquots, and the plate was cover with foil for 30 minutes at room temperature;
- Following 30 minutes, 50µl of Stop Solution was added to each well and the absorbance at 490nm was read from each well.

The absorbance values for each well was corrected in accordance with the manufacturer's instructions and the percentage of cytotoxicity was calculated using the following formula, normalised to empty vector transduced T cells, to give a fold change in cell killing. $% Cytotoxicity = \frac{Experimental - Effector Spontaneous - Target Spontaneous}{Target Maximum - Target Spontaneous} ∗ 100$

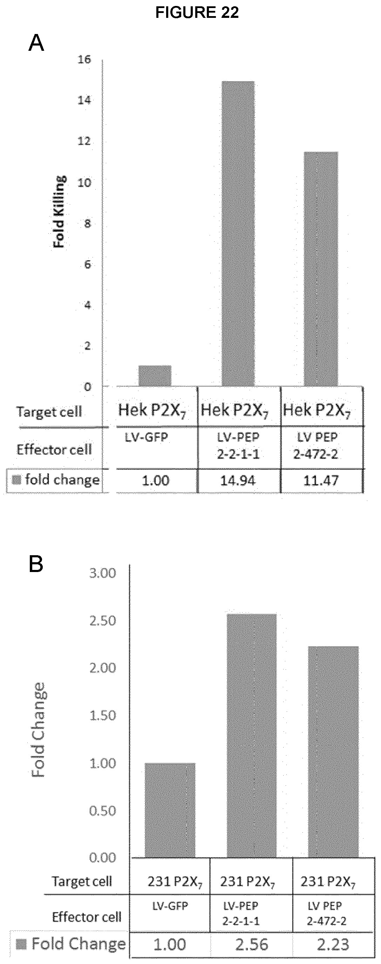

As demonstrated in Figure 22A, both PEP2-2-1-1 and PEP2-472-2 CAR expressing CD8 T cell killed approximately 15 and 11-fold (respectively) more HEK cells expressing a non-functional P2X₇ receptor than CD8 cells transduced with an empty vector. Furthermore, as shown in Figure 22B, PEP2-2-1-1 and PEP2-472-2 CAR expressing CD8 T cell killed approximately 2.5 and 2.25-fold (respectively) more 231 P2X₇ cells than CD8 cells transduced with an empty vector.

All methods described herein can be performed in any suitable order unless indicated otherwise herein or clearly contradicted by context. The use of any and all examples, or exemplary language (e.g., "such as") provided herein, is intended merely to better illuminate the example embodiments and does not pose a limitation on the scope of the claimed invention unless otherwise claimed. No language in the specification should be construed as indicating any non-claimed element as essential.

The description provided herein is in relation to several embodiments which may share common characteristics and features. It is to be understood that one or more features of one embodiment may be combinable with one or more features of the other embodiments. In addition, a single feature or combination of features of the embodiments may constitute additional embodiments.

The subject headings used herein are included only for the ease of reference of the reader and should not be used to limit the subject matter found throughout the disclosure or the claims. The subject headings should not be used in construing the scope of the claims or the claim limitations.

Those skilled in the art will appreciate that the invention described herein is susceptible to variations and modifications other than those specifically described. It is to be understood that the invention includes all such variations and modifications. The invention also includes all of the steps, features, compositions and compounds referred to, or indicated in this specification, individually or collectively, and any and all combinations of any two or more of the steps or features.

Also, it is to be noted that, as used herein, the singular forms "a", "an" and "the" include plural aspects unless the context already dictates otherwise.

Future patent applications may be filed on the basis of the present application, for example by claiming priority from the present application, by claiming a divisional status and/or by claiming a continuation status. It is to be understood that the following claims are not intended to limit the scope of what may be claimed in any such future application.

## Claims

1. A chimeric antigen receptor including:
an antigen-recognition domain that recognises a dysfunctional P2X₇ receptor, wherein the antigen-recognition domain recognises an epitope that includes amino acids 200 to 210 of the dysfunctional P2X₇ receptor corresponding to positions 200 to 210 of SEQ ID NO: 3 ;
a transmembrane domain; and
a signalling domain including an intracellular signalling portion of an activation receptor and/or an intracellular signalling portion of a co-stimulatory receptor.

2. The chimeric antigen receptor according to claim 1, wherein the antigen recognition domain recognises an epitope that includes amino acids 297 to 306 of the dysfunctional P2X₇ receptor corresponding to positions 297 to 306 of SEQ ID NO: 3.

3. The chimeric antigen receptor according to claim 1 or 2, wherein the antigen recognition domain comprises three CDRs of the amino acid sequence shown in SEQ ID NO: 10, 32, 33 or 34.

4. The chimeric antigen receptor according to any one of claim 1 to 3, wherein the antigen-recognition domain is a Fab, scFv, or sdAb.

5. The chimeric antigen receptor according to any one of claims 1 to 4, wherein the antigen-recognition domain is multivalent.

6. The chimeric antigen receptor according to claims 5, wherein the antigen-recognition domain is divalent or trivalent.

7. The chimeric antigen receptor according to any one of claims 1 to 6, wherein the antigen-recognition domain comprises:
• CDR1 comprising an amino acid sequence of residues 30 to 35 of SEQ ID NO: 10;
• CDR2 comprising an amino acid sequence of residues 50 to 67 of SEQ ID NO: 10; and
• CDR3 comprising an amino acid sequence of residues 98 to 108 of SEQ ID NO: 10.

8. The chimeric antigen receptor according to any one of claims 1 to 6, wherein the antigen-recognition domain comprises:
• CDR1 comprising an amino acid sequence of residues 30 to 35 of SEQ ID NO:32;
• CDR2 comprising an amino acid sequence of residues 50 to 67 of SEQ ID NO: 32; and
• CDR3 comprising an amino acid sequence of residues 98 to 108 of SEQ ID NO: 32.

9. The chimeric antigen receptor according to any one of claims 1 to 6, wherein the antigen-recognition domain comprises:
• CDR1 comprising an amino acid sequence of residues 30 to 35 of SEQ ID NO: 33;
• CDR2 comprising an amino acid sequence of residues 50 to 67 of SEQ ID NO: 33; and
• CDR3 comprising an amino acid sequence of residues 98 to 108 of SEQ ID NO: 33.

10. The chimeric antigen receptor according to any one of claims 1 to 6, wherein the antigen-recognition domain comprises:
• CDR1 comprising an amino acid sequence of residues 30 to 35 of SEQ ID NO: 34;
• CDR2 comprising an amino acid sequence of residues 50 to 67 of SEQ ID NO: 34; and
• CDR3 comprising an amino acid sequence of residues 98 to 108 of SEQ ID NO: 34.

11. The chimeric antigen receptor according to any one of claims 1 to 4, comprising the amino acid sequence of any one of SEQ ID NOs: 26, 27, 52, 53 or 54.

12. A nucleic acid molecule including a nucleotide sequence encoding the chimeric antigen receptor according to claim 1 to 11.

13. A viral vector including a nucleic acid according to claim 12, for viral transduction of a host cell.

14. A genetically modified cell, the cell including the chimeric antigen receptor according to of any one of claims 1 to 11, a nucleic acid molecule according to claim 12, or a viral vector according to claim 13, optionally wherein the cell is a leukocyte, a Peripheral Blood Mononuclear Cell (PBMC), a lymphocyte, a T cell, a CD4+ T cell, a CD8+ T cell, a natural killer cell or a natural killer T cell.

15. A genetically modified cell according to claim 14, for use in a method of killing a cell expressing a dysfunctional P2X₇ receptor, the method including exposing the cell expressing a dysfunctional P2X₇ receptor to the genetically modified cell.

16. The genetically modified cell for use according to claim 15, wherein the cell expressing a dysfunctional P2X₇ receptor is a cancer cell, optionally wherein the cancer cell is selected from one or more of; brain cancer, oesophageal cancer, mouth cancer, tongue cancer, thyroid cancer, lung cancer, stomach cancer, pancreatic cancer, kidney cancer, colon cancer, rectal cancer, prostate cancer, bladder cancer, cervical cancer, epithelial cell cancers, skin cancer, leukaemia, lymphoma, myeloma, breast cancer, ovarian cancer, endometrial cancer and testicular cancer, preferably wherein the cancer cell is selected from one or more of; lung cancer, oesophageal cancer, stomach cancer, colon cancer, prostate cancer, bladder cancer, cervical cancer, vaginal cancers, epithelial cell cancers, skin cancer, blood-related cancers, breast cancer, endometrial cancer, uterine cancer and testicular cancer.

17. A pharmaceutical composition including a genetically modified cell according to claim 14 and a pharmaceutically acceptable carrier.

## Patentansprüche

1. Chimärer Antigenrezeptor, einschließlich:
einer Antigenerkennungsdomäne, die einen dysfunktionalen P2X₇-Rezeptor erkennt, wobei die Antigenerkennungsdomäne ein Epitop, das Aminosäuren 200 bis 210 des dysfunktionalen P2X₇-Rezeptors entsprechend den Positionen 200 bis 210 von SEQ ID NO: 3 einschließt, erkennt;
einer Transmembrandomäne; und
einer Signaldomäne, einschließlich eines intrazellulären Signalabschnitts eines Aktivierungsrezeptors und/oder eines intrazellulären Signalabschnitts eines kostimulatorischen Rezeptors.

2. Chimärer Antigenrezeptor nach Anspruch 1, wobei die Antigenerkennungsdomäne ein Epitop, das Aminosäuren 297 bis 306 des dysfunktionalen P2X₇-Rezeptors entsprechend den Positionen 297 bis 306 von SEQ ID NO: 3 einschließt, erkennt.

3. Chimärer Antigenrezeptor nach Anspruch 1 oder 2, wobei die Antigenerkennungsdomäne drei CDRs der Aminosäuresequenz, die in SEQ ID NO: 10, 32, 33 oder 34 gezeigt werden, umfasst.

4. Chimärer Antigenrezeptor nach einem der Ansprüche 1 bis 3, wobei die Antigenerkennungsdomäne ein Fab, scFv oder sdAb ist.

5. Chimärer Antigenrezeptor nach einem der Ansprüche 1 bis 4, wobei die Antigenerkennungsdomäne mehrwertig ist.

6. Chimärer Antigenrezeptor nach Anspruch 5, wobei die Antigenerkennungsdomäne zweiwertig oder dreiwertig ist.

7. Chimärer Antigenrezeptor nach einem der Ansprüche 1 bis 6, wobei die Antigenerkennungsdomäne umfasst:
• CDR1, umfassend eine Aminosäuresequenz von Resten 30 bis 35 von SEQ ID NO: 10;
• CDR2 umfassend eine Aminosäuresequenz von Resten 50 bis 67 von SEQ ID NO: 10; und
• CDR3, umfassend eine Aminosäuresequenz von Resten 98 bis 108 von SEQ ID NO: 10.

8. Chimärer Antigenrezeptor nach einem der Ansprüche 1 bis 6, wobei die Antigenerkennungsdomäne umfasst:
• CDR1, umfassend eine Aminosäuresequenz von Resten 30 bis 35 von SEQ ID NO: 32;
• CDR2 umfassend eine Aminosäuresequenz von Resten 50 bis 67 von SEQ ID NO: 32; und
• CDR3, umfassend eine Aminosäuresequenz von Resten 98 bis 108 von SEQ ID NO: 32.

9. Chimärer Antigenrezeptor nach einem der Ansprüche 1 bis 6, wobei die Antigenerkennungsdomäne umfasst:
• CDR1, umfassend eine Aminosäuresequenz von Resten 30 bis 35 von SEQ ID NO: 33;
• CDR2 umfassend eine Aminosäuresequenz von Resten 50 bis 67 von SEQ ID NO: 33; und
• CDR3, umfassend eine Aminosäuresequenz von Resten 98 bis 108 von SEQ ID NO: 33.

10. Chimärer Antigenrezeptor nach einem der Ansprüche 1 bis 6, wobei die Antigenerkennungsdomäne umfasst:
• CDR1, umfassend eine Aminosäuresequenz von Resten 30 bis 35 von SEQ ID NO: 34;
• CDR2 umfassend eine Aminosäuresequenz von Resten 50 bis 67 von SEQ ID NO: 34; und
• CDR3, umfassend eine Aminosäuresequenz von Resten 98 bis 108 von SEQ ID NO: 34.

11. Chimärer Antigenrezeptor nach einem der Ansprüche 1 bis 4, umfassend die Aminosäuresequenz einer beliebigen einen von SEQ ID NOs: 26, 27, 52, 53 oder 54.

12. Nukleinsäuremolekül, einschließlich einer Nukleotidsequenz, die für den chimären Antigenrezeptor nach Anspruch 1 bis 11 codiert.

13. Viraler Vektor, einschließlich einer Nukleinsäure nach Anspruch 12, für virale Transduktion einer Wirtszelle.

14. Genetisch modifizierte Zelle, die Zelle einschließlich des chimären Antigenrezeptors nach einem der Ansprüche 1 bis 11, eines Nukleinsäuremoleküls nach Anspruch 12 oder eines viralen Vektors nach Anspruch 13, optional wobei die Zelle ein Leukozyt, eine mononukleäre Zelle peripheren Bluts (PBMC), ein Lymphozyt, eine T-Zelle, eine CD4+-T-Zelle, eine CD8+-T-Zelle, eine natürliche Killerzelle oder eine natürliche Killer-T-Zelle ist.

15. Genetisch modifizierte Zelle nach Anspruch 14 zur Verwendung in einem Verfahren zum Abtöten einer Zelle, die einen dysfunktionalen P2X₇-Rezeptor exprimiert, das Verfahren einschließlich eines Aussetzens der Zelle, die einen dysfunktionalen P2X₇-Rezeptor exprimiert, der genetisch modifizierten Zelle.

16. Genetisch modifizierte Zelle zur Verwendung nach Anspruch 15, wobei die Zelle, die einen dysfunktionalen P2X₇-Rezeptor exprimiert, eine Krebszelle ist, optional wobei die Krebszelle ausgewählt ist aus einem oder mehreren von; Hirnkrebs, Speiseröhrenkrebs, Mundkrebs, Zungenkrebs, Schilddrüsenkrebs, Lungenkrebs, Magenkrebs, Bauchspeicheldrüsenkrebs, Nierenkrebs, Dickdarmkrebs, Mastdarmkrebs, Prostatakrebs, Blasenkrebs, Gebärmutterhalskrebs, Epithelzellkrebserkrankungen, Hautkrebs, Leukämie, Lymphom, Myelom, Brustkrebs, Eierstockkrebs, Endometriumkrebs und Hodenkrebs, vorzugsweise wobei die Krebszelle ausgewählt ist aus einem oder mehreren von; Lungenkrebs, Speiseröhrenkrebs, Magenkrebs, Dickdarmkrebs, Prostatakrebs, Blasenkrebs, Gebärmutterhalskrebs, Vaginalkrebserkrankungen, Epithelzellkrebserkrankungen, Hautkrebs, blutbedingten Krebserkrankungen, Brustkrebs, Endometriumkrebs, Gebärmutterkrebs und Hodenkrebs.

17. Pharmazeutische Zusammensetzung, einschließlich einer genetisch modifizierten Zelle nach Anspruch 14 und einer pharmazeutisch verträglichen Trägersubstanz.

## Revendications

1. Récepteur antigénique chimérique comportant :
un domaine de reconnaissance d'antigène qui reconnaît un récepteur P2X₇ dysfonctionnel, dans lequel le domaine de reconnaissance d'antigène reconnaît un épitope qui comporte les acides aminés 200 à 210 du récepteur P2X₇ dysfonctionnel correspondant aux positions 200 à 210 de SEQ ID NO : 3 ;
un domaine transmembranaire ; et
un domaine de signalisation comportant une partie de signalisation intracellulaire d'un récepteur d'activation et/ou une partie de signalisation intracellulaire d'un récepteur de costimulation.

2. Récepteur antigénique chimérique selon la revendication 1, dans lequel le domaine de reconnaissance d'antigène reconnaît un épitope qui comporte les acides aminés 297 à 306 du récepteur P2X₇ dysfonctionnel correspondant aux positions 297 à 306 de SEQ ID NO : 3.

3. Récepteur antigénique chimérique selon la revendication 1 ou 2, dans lequel le domaine de reconnaissance d'antigène comprend trois CDR de la séquence d'acides aminés présentée dans SEQ ID NO : 10, 32, 33 ou 34.

4. Récepteur antigénique chimérique selon l'une quelconque des revendications 1 à 3, dans lequel le domaine de reconnaissance d'antigène est un Fab, un scFv, ou un sdAb.

5. Récepteur antigénique chimérique selon l'une quelconque des revendications 1 à 4, dans lequel le domaine de reconnaissance d'antigène est multivalent.

6. Récepteur antigénique chimérique selon les revendications 5, dans lequel le domaine de reconnaissance d'antigène est divalent ou trivalent.

7. Récepteur antigénique chimérique selon l'une quelconque des revendications 1 à 6, dans lequel le domaine de reconnaissance d'antigène comprend :
• CDR1 comprenant une séquence d'acides aminés des résidus 30 à 35 de SEQ ID NO : 10 ;
• CDR2 comprenant une séquence d'acides aminés des résidus 50 à 67 de SEQ ID NO : 10 ; et
• CDR3 comprenant une séquence d'acides aminés des résidus 98 à 108 de SEQ ID NO : 10.

8. Récepteur antigénique chimérique selon l'une quelconque des revendications 1 à 6, dans lequel le domaine de reconnaissance d'antigène comprend :
• CDR1 comprenant une séquence d'acides aminés des résidus 30 à 35 de SEQ ID NO : 32 ;
• CDR2 comprenant une séquence d'acides aminés des résidus 50 à 67 de SEQ ID NO : 32 ; et
• CDR3 comprenant une séquence d'acides aminés des résidus 98 à 108 de SEQ ID NO : 32.

9. Récepteur antigénique chimérique selon l'une quelconque des revendications 1 à 6, dans lequel le domaine de reconnaissance d'antigène comprend :
• CDR1 comprenant une séquence d'acides aminés des résidus 30 à 35 de SEQ ID NO : 33 ;
• CDR2 comprenant une séquence d'acides aminés des résidus 50 à 67 de SEQ ID NO : 33 ; et
• CDR3 comprenant une séquence d'acides aminés des résidus 98 à 108 de SEQ ID NO : 33.

10. Récepteur antigénique chimérique selon l'une quelconque des revendications 1 à 6, dans lequel le domaine de reconnaissance d'antigène comprend :
• CDR1 comprenant une séquence d'acides aminés des résidus 30 à 35 de SEQ ID NO : 34 ;
• CDR2 comprenant une séquence d'acides aminés des résidus 50 à 67 de SEQ ID NO : 34 ; et
• CDR3 comprenant une séquence d'acides aminés des résidus 98 à 108 de SEQ ID NO : 34.

11. Récepteur antigénique chimérique selon l'une quelconque des revendications 1 à 4, comprenant la séquence d'acides aminés de l'une quelconque des SEQ ID NO : 26, 27, 52, 53 ou 54.

12. Molécule d'acide nucléique comportant une séquence nucléotidique codant pour le récepteur antigénique chimérique selon la revendication 1 à 11.

13. Vecteur viral comportant un acide nucléique selon la revendication 12, pour une transduction virale d'une cellule hôte.

14. Cellule génétiquement modifiée, la cellule comportant le récepteur antigénique chimérique selon l'une quelconque des revendications 1 à 11, une molécule d'acide nucléique selon la revendication 12, ou un vecteur viral selon la revendication 13, facultativement dans laquelle la cellule est un leucocyte, une cellule mononucléée du sang périphérique (PBMC), un lymphocyte, un lymphocyte T, un lymphocyte T CD4+, un lymphocyte T CD8+, une cellule tueuse naturelle ou un lymphocyte T tueur naturel.

15. Cellule génétiquement modifiée selon la revendication 14, pour une utilisation dans un procédé de destruction d'une cellule exprimant un récepteur P2X₇ dysfonctionnel, le procédé comportant l'exposition de la cellule exprimant un récepteur P2X₇ dysfonctionnel à la cellule génétiquement modifiée.

16. Cellule génétiquement modifiée pour une utilisation selon la revendication 15, dans laquelle la cellule exprimant un récepteur P2X₇ dysfonctionnel est une cellule cancéreuse, facultativement dans laquelle la cellule cancéreuse est choisie parmi un ou plusieurs parmi ; cancer du cerveau, cancer de l'œsophage, cancer de la bouche, cancer de la langue, cancer de la thyroïde, cancer du poumon, cancer de l'estomac, cancer du pancréas, cancer du rein, cancer du côlon, cancer du rectum, cancer de la prostate, cancer de la vessie, cancer du col de l'utérus, cancers des cellules épithéliales, cancer de la peau, leucémie, lymphome, myélome, cancer du sein, cancer de l'ovaire, cancer de l'endomètre et cancer du testicule, de préférence dans laquelle la cellule cancéreuse est choisie parmi un ou plusieurs parmi ; cancer du poumon, cancer de l'œsophage, cancer de l'estomac, cancer du côlon, cancer de la prostate, cancer de la vessie, cancer du col de l'utérus, cancers du vagin, cancers des cellules épithéliales, cancer de la peau, cancers liés au sang, cancer du sein, cancer de l'endomètre, cancer de l'utérus et cancer du testicule.

17. Composition pharmaceutique comportant une cellule génétiquement modifiée selon la revendication 14 et un véhicule pharmaceutiquement acceptable.
